# EUROPEAN PATENT APPLICATION

(11) **EP 2 407 558 A1**
(43) Date of publication of application: **18.01.2012**
(21) Application number: 11005913.6
(22) Date of filing: 31.10.2007
(51) Int. Cl.: C12Q 1/68

(54) **Methods for the detection of a single- or double-stranded nucleic acid molecule**

(30) Priority: 31.10.2006 EP 06022735
(62) Divisional of application: 07819505.4
(71) Applicant: Noxxon Pharma AG, 10589 Berlin (DE)
(72) Inventor: Jarosch, Florian, 13469 Berlin (DE); Hansen, Hilke, 24960 Munkbrarup (DE); Lange, Christian, 13187 Berlin (DE)
(74) Representative: Bohmann, Armin K.

(57) **Abstract**

The present invention is related to a method for the detection of a nucleic acid molecule comprising at least a strand comprising a sequence of nucleotides in a sample, whereby the method comprises the following steps:
a) providing a sample containing the nucleic acid molecule;
b) providing a capture probe, whereby the capture probe is at least partially complementary to a part of the nucleic acid molecule;
c) allowing the capture probe to react with the nucleic acid molecule or a part thereof; and
d) detecting whether or not the capture probe is hybridized to the nucleic acid molecule or part thereof,

wherein the capture probe comprises a first capture probe and a second capture probe, whereby the first capture probe is at least partially complementary to a first part of said a strand of the nucleic acid molecule and the second capture probe is at least partially complementary to a second part of said a strand of the nucleic acid molecule.

## Description

The present invention is related to methods for the detection of a single- or double-stranded nucleic acid molecule in a sample and a kit for such method.

RNA-mediated interference (RNAi) is a post-transcriptional gene silencing mechanism initiated by double stranded RNA (dsRNA) homologous in sequence to the silenced gene (Fire (1999), Trends Genet 15, 358-63; Tuschl, et al. (1999), Genes Dev 13, 3191-7; Waterhouse, et al. (2001), Nature 411, 834-42; Elbashir, et al. (2001), Nature 411, 494-8; for review see Sharp (2001), Genes Dev 15, 485-90 and Barstead (2001), Curr Opin Chem Biol 5, 63-6). RNAi has been used extensively to determine gene function in a number of organisms, including plants (Baulcombe (1999), Curr Opin Plant Biol 2, 109-13), nematodes (Montgomery, et al. (1998), Proc Natl Acad Sci U S A 95, 15502-7), *Drosophila* (Kennerdell, et al. (1998), Cell 95, 1017-26; Kennerdell, et al. (2000), Nat Biotechnol 18, 896-8). In the nematode *C*. *elegans* about one third of the genome has already been subjected to functional analysis by RNAi (Kim (2001), Curr Biol 11, R85-7; Maeda, et al. (2001), Curr Biol 11, 171-6).

Until recently RNAi in mammalian cells was not generally applicable, with the exception of early mouse development (Wianny, et al. (2000), Nat Cell Biol 2, 70-5). The discovery that transfection of duplexes of 21-nt RNA into mammalian cells interfered with gene expression and did not induce a sequence independent interferon-driven anti-viral response usually obtained with long dsRNA led to new potential application in differentiated mammalian cells (Elbashir et al. (2001), Nature 411, 494-8). Interestingly these small interfering RNAs (siRNAs) resemble the processing products from long dsRNAs suggesting a potential bypassing mechanism in differentiated mammalian cells. The Dicer complex, a member of the RNAse III family, necessary for the initial dsRNA processing has been identified (Bernstein, et al. (2001), Nature 409, 363-6; Billy, et al. (2001), Proc Natl Acad Sci U S A 98, 14428-33). One of the problems previously encountered when using unmodified ribooligonucleotides was the rapid degradation in cells or even in the serum-containing medium (Wickstrom (1986), J Biochem Biophys Methods 13, 97-102; Cazenave, et al. (1987), Nucleic Acids Res 15, 10507-21). It will depend on the particular gene function and assay systems used whether the respective knock down induced by transfected siRNA will be maintained long enough to achieve a phenotypic change.

Such as siRNAs, ribozymes and antisense oligonucleotides have in common that they address the same target molecule, namely the messenger RNA (mRNA) of the gene product to be suppressed.

Antisense oligonucleotides are specific single-stranded nucleic acids that bind to the mRNA strand, by what mRNA is blocked for the transcription of the mRNA into the gene product. Moreover the mRNA is degraded by RNAseH digestion (Scherer and Rossi (2003), Nature Biotechnology 21: 1457-1465). Originally antisense oligonucleotides were composed of D-nucleic acids like RNA or DNA. Alternative to RNA and DNA antisense oligonucleotides can be composed of peptide nucleic acids (PNA), locked nucleic acids (LNA) or morpholino oligonucleotides (Karkare and Bhatnagar (2006) Appl Microbiol Biotechnol.71(5):575-86).

Peptide nucleic acid is a chemical similar to DNA or RNA. DNA and RNA have a deoxyribose and ribose sugar backbone, respectively, whereas PNA's backbone is composed of repeating N-(2-aminoethyl)-glycine units linked by peptide bonds. The various purine and pyrimidine bases are linked to the backbone by methylene carbonyl bonds. PNAs are depicted like peptides, with the N-terminus at the first (left) position and the C-terminus at the right. Since the backbone of PNA contains no charged phosphate groups, the binding between PNA/DNA strands is stronger than between DNA/DNA strands due to the lack of electrostatic repulsion. Early experiments with homopyrimidine strands (strands consisting of only one repeated pyrimidine base) have shown that the Tm ("melting" temperature) of a 6-base thymine PNA/adenine DNA double helix was 31 °C in comparison to an equivalent 6-base DNA/DNA duplex that denatures at a temperature less than 10°C. Mixed base PNA molecules are true mimics of DNA molecules in terms of base-pair recognition. PNA/PNA binding is stronger than PNA/DNA binding. Synthetic peptide nucleic acid oligomers have been used in recent years in molecular biology procedures, diagnostic assays and antisense therapies. Due to their higher binding strength it is not necessary to design long PNA oligomers for use in these roles, which usually require oligonucleotide probes of 20-25 bases. The main concern of the length of the PNA-oligomers is to guarantee the specificity. PNA oligomers also show greater specificity in binding to complementary DNAs, with a PNA/DNA base mismatch being more destabilizing than a similar mismatch in a DNA/DNA duplex. This binding strength and specificity also applies to PNA/RNA duplexes. PNAs are not easily recognized by either nucleases or proteases, making them resistant to enzyme degradation. PNAs are also stable over a wide pH range (Karkare and Bhatnagar (2006) Appl Microbiol Biotechnol.71(5):575-86; Marin et al. (2004) Expert Opin Biol Ther. 4(3):337-48; Nielsen (1999) Current Opnion in Biotechnology 10:71-75).

Locked nucleic acid (LNA), often referred to as inaccessible RNA, is a modified RNA nucleic acid. The ribose moiety of an LNA nucleotide is modified with an extra bridge connecting the 2' and 4' carbons. The bridge "locks" the ribose in the 3'-endo structural conformation, which is often found in the A-form of DNA or RNA. LNA nucleotides can be mixed with DNA or RNA bases in the oligonucleotide whenever desired. The locked ribose conformation enhances base stacking and backbone pre-organization. This significantly increases the thermal stability (melting temperature) of oligonucleotides (Petersen and Wengel (2003) Trends Biotechnol.21(2):74-81.). Therefore antisense oligonucleotides composed of LNA were used for gene silencing in vitro and in vivo (Gruenweller and Hartmann (2007) BioDrugs. 21(4):235-43).

Morpholino antisense oligonucleotides are used to modify gene expression and to block access of other molecules to specific sequences within a nucleic acid. Morpholinos are synthetic molecules which are the product of a redesign of natural nucleic acid structure (Summerton and Weller (1997) Antisense & Nucleic Acid Drug Development 7: 187-95). Usually 25 bases in length, they bind to complementary sequences of RNA by standard nucleic acid base-pairing. Structurally, the difference between Morpholinos and DNA is that while Morpholinos have standard nucleic acid bases, those bases are bound to morpholine rings instead of deoxyribose rings and linked through phosphorodiamidate groups instead of phosphates (Summerton and Weller (1997) Antisense & Nucleic Acid Drug Development 7: 187-95). Morpholinos are in development as pharmaceutical therapeutics, e.g. targeted against pathogenic organisms such as bacteria (Geller (2005) Curr. Opin. Mol. Ther. 7 (2): 109-13) or viruses (Deas et al (2007) Antimicrob Agents Chemother. 51(7):2470-82) and for amelioration of genetic diseases (McClorey et al. (2006) Neuromuscul Disord. 16 (9-10): 583-90).

Ribozymes are D-nucleic acids that catalyzes a chemical reaction. Many natural ribozymes catalyze either their own cleavage or the cleavage of other RNAs, e.g. mRNAs. Ribozymes bind the mRNA strand and cleaves it specifically. By this cleavage or degradation of the target-specific mRNA molecule, the expression of the target molecule is avoided (Usman and Blatt (2000), Journal of Clinical Investigation, 106: 1197-1202).

Furthermore single-stranded nucleic acids can form distinct and stable three-dimensional structures and specifically bind to a target molecules like antibodies. Such nucleic acids composed of D-nucleotides are called aptamers. Aptamers can be identified against several target molecules, e.g. small molecules, proteins, nucleic acids, and even cells, tissues and organisms) and can inhibit the in vitro and/or in vivo function of the specific target molecule. Aptamers are usually identified by a target-directed selection process, called *in vitro* selection or SELEX (systematic evolution of ligands by exponential enrichment) (Ellington and Szostak (1990), Nature,;346(6287):818-22; Bock et al. (1992), Nature, 355(6360):564-6; Tuerck and Gold (1990), Science 249:505-510). Non-modified aptamers are cleared rapidly from the bloodstream, with a half-life of minutes to hours, mainly due to nuclease degradation and clearance from the body by the kidneys, a result of the aptamer's inherently low molecular weight. Hence, in order to use aptamers therapeutically they have to be modified at the 2' position of the sugar (e.g. ribose) backbone (Cload et al. (2006). Properties of Therapeutic Aptamers.. In The Aptamer Handbook, S. Klussmann, ed. (Weinheim, Wiley-VCH), pp. 417-442.)

The omnipresent nucleases which account for the instability of aptamers consist of chiral building blocks, i.e. L-amino acids. Consequently, the structure of nucleases is inherently chiral as well, resulting in stereospecific substrate recognition. Hence, these enzymes only accept substrate molecules in the adequate chiral configuration. Since aptamers and naturally occurring nucleic acids are composed of D-nucleotides, an L-oligonucleotide should escape from enzymatic recognition and subsequent degradation. Due to the same principle, unfortunately in this case, nature developed no enzymatic activity to amplify such mirror-image nucleic acids. Accordingly, L-nucleic acid aptamers cannot be directly obtained employing the SELEX process. The principles of stereochemistry, though, reveal a detour which eventually leads to the desired functional L-nucleic acid aptamers.

If an in vitro selected (D-)aptamer binds its natural target, the structural mirror-image of this aptamer binds with the same characteristics the mirror-image of the natural target. Here, both interaction partners have the same (unnatural) chirality. Due to the homochirality of life and most biochemical compounds, such enantio-RNA ligands, of course, would be of limited practical use. If, on the other hand, the SELEX process is carried out against an (unnatural) mirror-image target, an aptamer recognizing this (unnatural) target will be obtained. The corresponding mirror-image configuration of said aptamer - the desired L-aptamer - in turn recognizes the natural target. This mirror-image selection process for the generation of biostable oligonucleotides was published first in 1996 (Nolte et al (1996), Nature Biotechnology (1996) 14(9):1116-9; Klussmann et al. (1996) Nature Biotechnololgy, 14(9):1112-5) and results in the generation of functional mirror-image oligonucleotide ligands that display not only high affinity and specificity for a given target molecule, but at the same time also biological stability. Such ligand-binding L-oligonucleotides were named 'Spiegelmers' (from the German word 'Spiegel', mirror) (Eulberg et al (2006), Spiegelmers for Therapeutic Applications -- Use of Chiral Principles in Evolutionary Selection Techniques. In The Aptamer Handbook, S. Klussmann, ed. (Weinheim, Wiley-VCH), pp. 417-442).

As alterations in gene expression have become a better understood component of normal development and disease pathogenesis, transcription factors and other regulators of gene expression have become an increasingly attractive target for potential therapeutic intervention. Transcription factors are generally nuclear proteins that play a critical role in gene regulation and can exert either a positive or negative effect on gene expression. These regulatory proteins bind specific sequences found in the promoter regions of their target genes. These binding sequences are generally 6-10 bp in length and are occasionally found in multiple iterations. Because transcription factors can recognize their relatively short binding sequences even in the absence of surrounding genomic DNA, short radiolabeled oligodeoxynucleotides (ODNs) bearing consensus binding sites can serve as probes in electrophoretic mobility shift assays, which identify and quantify transcription factor binding activity in nuclear extracts. More recently, ODNs bearing the consensus binding sequence of a specific transcription factor have been explored as tools for manipulating gene expression in living cells. This strategy involves the intracellular delivery of such "decoy" ODNs, which are then recognized and bound by the target factor. Occupation of the transcription factor's DNA-binding site by the decoy renders the protein incapable of subsequently binding to the promoter regions of target genes (Mann and Dzau (2000) J Clin Invest. 106(9):1071-5). The use of decoy ODNs for the therapeutic manipulation of gene expression was firstly described by .Morishita et al.. They reported the treatment of rat carotid arteries at the time of balloon injury with ODNs bearing the consensus binding site for the E2F family of transcription factors and found that a decoy specific to E2F-1 prevented this upregulation and blocked smooth muscle proliferation and neointimal hyperplasia in injured vessels (Morishita et al. (1995) Proc Natl Acad Sci USA 92:5855-5859). In addition to this initial in vivo application, a transcription factor decoy was used to block a negative regulatory element in the promoter of the Renin gene in the mouse submandibular gland, demonstrating that decoys can be used to increase as well as to suppress gene activity in vivo (Yamada et al. (1995) J Clin Invest. 96:1230-1237; Tomita et al. (1999) Circ Res. 84:1059-1066).

The potency of RNA interference mediating molecules, antisense oligonucleotides, ribozymes, aptamers, Spiegelmers and decoy oligonucleotides in modulation of the effects of molecules that are involved in various diseases has recently led to the development of therapeutic RNA interference mediating molecules, therapeutic antisense oligonucleotides, therapeutic ribozymes, therapeutic decoy oligonucleotides, therapeutic aptamers and therapeutic Spiegelmers (Eckstein (2007) Expert Opin Biol Ther. 7(7):1021-34; Cload et al. (2006). Properties of Therapeutic Aptamers.. In The Aptamer Handbook, S. Klussmann, ed. (Weinheim, Wiley-VCH), pp. 417-442; Eulberg et al (2006), Spiegelmers for Therapeutic Applications -- Use of Chiral Principles in Evolutionary Selection Techniques. In The Aptamer Handbook, S. Klussmann, ed. (Weinheim, Wiley-VCH), pp. 417-442; (Mann and Dzau (2000) J Clin Invest. 106(9):1071-5). Particularly with regard to the testing of the respective, potentially therapeutically active compounds, there is a need for detecting and, in a sub-aspect thereof, quantifying such RNAi mediating molecules, antisense oligonucleotides, ribozymes, decoy oligonucleotides, aptamers and Spiegelmers, in a sample.

Therefore, the problem underlying the present invention was to provide for a method for the detection in a sample of a nucleic acid molecule, more preferably a single- or double-stranded nucleic acid molecule whereby the double-stranded nucleic acid molecule comprises a first strand and a second strand, whereby the first strand and the second strand are essentially complementary to each other.

A further problem was to provide for a kit which allows the performance of such method.

These and other problems are solved by the subject matter of the independent embodiments attached hereto, whereby preferred embodiments may be taken from the dependent embodiments.

More specifically, in a first aspect the problem underlying the present invention is solved by a method for the detection of a nucleic acid molecule comprising at least a strand comprising a sequence of nucleotides in a sample, whereby the method comprises the following steps:
a) providing a sample containing the nucleic acid molecule;
b) providing a capture probe, whereby the capture probe is at least partially complementary to a part of the nucleic acid molecule;
c) allowing the capture probe to react with the nucleic acid molecule or a part thereof; and
d) detecting whether or not the capture probe is hybridized to the nucleic acid molecule or part thereof.

In an embodiment of the first aspect in step c) a complex is formed consisting of the nucleic acid molecule and the capture probe.

In an embodiment of the first aspect the capture probe comprises a detection means which allows the detection of the capture probe.

In a preferred embodiment of the first aspect after step c) a nuclease activity is added to the reaction, whereby the nuclease activity is degrading the capture probe which is not hybridized to the nucleic acid molecule.

In an embodiment of the first aspect the capture probe is a single- stranded nucleic acid, preferably a single-stranded ribonucleic acid, and the nuclease activity is a single-stranded specific RNAse.

In an embodiment of the first aspect the nucleic acid molecule is hybridizing to the capture probe over the entire length of said a strand of the nucleic acid molecule.

In a preferred embodiment of the first aspect the complex consisting of the capture probe and said a strand of the nucleic acid molecule forms a blunt end or a protruding 3' and/ or 5'-end of the capture probe.

In an embodiment of the first aspect the capture probe is immobilized to a support, preferably a solid support, by the 5' end of the capture probe.

In an embodiment of the first aspect the capture probe is immobilized to a support, preferably a solid support, by the 3' end of the capture probe.

In an embodiment of the first aspect the nucleic acid molecule is a single-stranded nucleic acid molecule.

In a preferred embodiment of the first aspect the single-stranded nucleic acid molecule consists of RNA, DNA, modified RNA, modified DNA, LNA or PNA, or combinations thereof, preferably DNA, RNA, modified RNA or modified DNA.

In an embodiment of the first aspect the nucleic acid molecule is a double-stranded nucleic acid molecule comprising a first strand and a second strand, and wherein the strand of the nucleic acid molecule according the first aspect of the present invention is the first strand or the second strand of the double-stranded nucleic acid, or a part thereof.

In a preferred embodiment of the first aspect the first strand or the second strand of the double-stranded nucleic acid molecule consists each and independently from each other of RNA, DNA, modified RNA or modified DNA, or combinations thereof.

In an embodiment of the first aspect prior, during or after step a) but before step c) the double-stranded molecule is separated into a first strand and a second strand.

In an embodiment of the first aspect in step d) it is detected whether the capture probe is hybridized to the first strand or the second strand.

In an embodiment of the first aspect the capture probe comprises a first capture probe and a second capture probe, whereby the first capture probe is at least partially complementary to a first part of said a strand of the nucleic acid molecule and the second capture probe is at least partially complementary to a second part of said a strand of the nucleic acid molecule.

In a preferred embodiment of the first aspect in step c) a complex is formed consisting of said a strand of the nucleic acid molecule and the first and the second capture probe.

In an embodiment of the first aspect either the first capture probe or the second capture probe comprises a detection means, and whereby either the second capture probe or the first capture probe can be immobilized to a support.

In a preferred embodiment of the first aspect the first or the second capture probe is immobilized to a support, preferably a solid support, by the 5' end of the first or second capture probe.

In an embodiment of the first aspect the first or second capture probe is immobilized to a support, preferably a solid support, by the 3' end of the first or second capture probe.

In an embodiment of the first aspect the first capture probe comprises the detection means and the second capture probe is immobilized to the surface and the molecules of the first capture probe that are not part of the complex are removed from the reaction so that in step d) only the molecules of the first capture probe that are part of the complex are detected
or
wherein the second capture probe comprises the detection means and the first capture probe is immobilized to the surface and the molecules of the second capture probe that are not part of the complex are removed from the reaction so that in step d) only the molecules of the second capture probe that are part of the complex are detected.

In an embodiment of the first aspect the nucleic acid molecule is a single-stranded nucleic acid molecule.

In a preferred embodiment of the first aspect the single-stranded molecule consists of RNA, DNA, modified RNA, modified DNA, LNA or PNA, or combinations thereof, preferably DNA, RNA, modified RNA or modified DNA.

In a preferred embodiment of the first aspect the single-stranded molecule consists of L-RNA or L-DNA, modified L-RNA, modified L-DNA or L-LNA, or combinations thereof, preferably L-DNA and L-RNA.

In an embodiment of the first aspect the nucleic acid molecule is a double-stranded nucleic acid comprising a first strand and a second strand, and wherein the strand of the nucleic acid molecule according to the first aspect of the present invention is the first strand or the second strand of the double-stranded nucleic acid, or a part thereof.

In a proffered embodiment of the first aspect the first strand or the second strand of the double-stranded nucleic acid consists each and independently from each other of RNA, DNA, modified RNA, modified DNA, or combinations thereof.

In an embodiment of the first aspect prior, during or after step a) but before step c) the double-stranded molecule is separated into a first strand and a second strand.

In an embodiment of the first aspect in step d) it is detected whether the capture probe is hybridized to the first strand or the second strand.

In a second aspect the problem underlying the present invention is solved by a method for the detection of a nucleic acid molecule comprising at least a strand comprising a sequence of nucleotides in a sample, whereby the method comprises the following steps:
a) providing a sample containing the nucleic acid molecule;
b) providing a capture probe, whereby the capture probe is at least partially complementary to a part of the nucleic acid molecule;
c) providing a detection probe comprising a detection means, whereby the detection probe is at least partially complementary to the capture probe;
d) allowing the capture probe and the detection probe to react either simultaneously or in any order sequentially with the nucleic acid molecule or a part thereof; and
e) detecting whether or not the capture probe is hybridized to the nucleic acid molecule.

In an embodiment of the second aspect the nucleic acid molecule comprises a nucleotide sequence which is at least partially complementary to the capture probe.

In a preferred embodiment of the second aspect in step e) it is detected whether the capture probe is hybridized to the nucleotide sequence of the nucleic acid molecule which is complementary to the capture probe.

In an embodiment of the second aspect step e) comprises the step of comparing the signal generated by the detection means when the capture probe and the detecting probe are hybridized in the presence of the nucleic acid molecule or part thereof, and in the absence of the nucleic acid molecule or part thereof.

In an embodiment of the second aspect the nucleic acid molecule is a single-stranded nucleic acid molecule.

In a preferred embodiment of the second aspect the single-stranded molecule consists of RNA, DNA, modified RNA, modified DNA, LNA or PNA, or combinations thereof, preferably DNA, RNA, modified RNA or modified DNA.

In a preferred embodiment of the second aspect the single-stranded molecule consists of L-RNA or L-DNA, modified L-RNA, modified L-DNA or L-LNA, or combinations thereof, preferably L-DNA and L-RNA.

In an embodiment of the second aspect the nucleic acid molecule is a double-stranded nucleic acid comprising a first strand and a second strand, and wherein the strand of the nucleic acid molecule according to the second aspect of the present invention is the first strand or the second strand of the double-stranded nucleic acid, or a part thereof.

In a preferred embodiment of the second aspect the first strand or the second strand of the double-stranded nucleic acid consists each and independently from each other of RNA, DNA, modified RNA, modified DNA, or combinations thereof.

In an embodiment of the second aspect prior, during or after step a) but before step c) the double-stranded molecule is separated into a first strand and a second strand.

In a third aspect the problem underlying the present invention is solved by a method for the detection of a nucleic acid molecule comprising at least a strand comprising a sequence of nucleotides in a sample, whereby the method comprises the following steps:
a) providing a sample containing the nucleic acid molecule;
   whereby a strand of the nucleic acid molecule consists of a first part and a second part;
b) providing a capture probe;
c) providing a first bridging probe, whereby a first part of the first bridging probe is at least partially complementary to the capture probe and a second part of the first bridging probe is at least partially complementary to the first part of said a strand of the nucleic acid and whereby when the second part of the bridging probe is hybridized to the first part of said a strand of the nucleic acid, the second part of the strand forms an overhang;
d) providing a second bridging probe comprising a first part and a second part, whereby the first part of the second bridging probe is at least partially complementary to the second part of said a strand of the nucleic acid, and allowing the second bridging probe to hybridise to the overhang formed in step c);
e) providing a detection probe, whereby the detection probe is at least partially complementary to the second part of the second bridging probe and comprises a detection means, and allowing the detection probe to hybridize to the second part of the second bridging probe;
f) ligating one end of said a strand of the nucleic acid to one end of the detection probe and/or the other end of the respective strand to one end of the capture probe; and
g) detecting whether or not the capture probe and/or the detection probe is/are ligated to said a strand of the nucleic acid provided in step a).

In an embodiment of the third aspect the ligation between the terminal nucleotide of the capture probe and the terminal nucleotide of said a first part of the strand of the nucleic acid molecule occurs under the proviso that the terminal nucleotide of the capture probe and the terminal nucleotide of the first part of said a strand of the nucleic acid molecule are immediately adjacent to each other.

In an embodiment of the third aspect the capture probe and the first part of said a strand of the nucleic acid molecule, taken together, form a nucleotide sequence which has about the same length and/or which comprises a nucleotide sequence which is essentially complementary to the first bridging probe or an part thereof consisting of adjacent nucleotides.

In an embodiment of the third aspect the ligation between the terminal nucleotide of the detection probe and the terminal nucleotide of the second part of said a strand of the nucleic acid molecule occurs under the proviso that the terminal nucleotide of the detection probe and the terminal nucleotide of the second part of said a strand of the nucleic acid molecule are immediately adjacent to each other.

In an embodiment of the third aspect the detection probe and the second part of said a strand of the nucleic acid molecule, taken together, form a nucleotide sequence which has at least the sequence of the second bridging probe and/or which comprises a nucleotide sequence which is essentially complementary to the second bridging probe or a part thereof consisting of adjacent nucleotides.

In an embodiment of the third aspect, after the ligation, the detection probe not ligated to said a strand of the nucleic acid molecule and/or said a strand of the nucleic acid molecule not ligated to the capture probe is/are removed from the reaction.

In an embodiment of the third aspect the capture probe is immobilized to a support, preferably a solid support, by the 3' end of the capture probe.

In a preferred embodiment of the third aspect the capture probe provides for a 5' end and/or said a strand of the nucleic acid molecule immobilized to the first bridging probe provides for a 5' end, whereby one or both of the 5'ends are monophosphorylated.

In an embodiment of the third aspect the capture probe is immobilized to a support, preferably a solid support, by the 5' end of the capture probe.

In a preferred embodiment of the third aspect the detection probe provides for a 5' end and/or said a strand of the nucleic acid molecule immobilized to the first bridging probe provides for a 5' end, whereby the one or both of the 5'ends are monophosphorylated.

In an embodiment of the third aspect the first bridging probe and the second bridging probe are a single molecule.

In a preferred embodiment of the third aspect the single molecule is created prior or subsequently to the addition of both the first bridging probe and the second bridging probe to the reaction, preferably by a ligase activity.

In an embodiment of the third aspect the stretch of nucleotides of the capture probe interacting with the first bridging probe has a length of about 2 to 20 consecutive nucleotides, preferably 6 to 15 consecutive nucleotides.

In an embodiment of the third aspect the first part of the first bridging probe has a length of about 2 to 20 consecutive nucleotides, preferably 6 to 15 consecutive nucleotides.

In an embodiment of the third aspect the second part of the first bridging probe has a length of about 2 to 20 consecutive nucleotides, preferably 7 to 11 consecutive nucleotides.

In an embodiment of the third aspect the detection probe comprises a length of about 2 to 20 consecutive nucleotides, preferably about 7 to 11 consecutive nucleotides.

In an embodiment of the third aspect the nucleic acid molecule is a single-stranded nucleic acid molecule.

In a preferred embodiment of the third aspect the single-stranded molecule consists of RNA, DNA, modified RNA or modified DNA, preferably DNA, RNA, modified RNA or modified DNA.

In an embodiment of the third aspect the nucleic acid molecule is a double-stranded nucleic acid molecule comprising a first strand and a second strand, and wherein the strand of the nucleic acid molecule according to the third aspect of the present invention is the first strand or the second strand of the double-stranded nucleic acid, or a part thereof.

In a preferred embodiment of the third aspect the first strand or the second strand of the double-stranded nucleic acid consists each and independently from each other of RNA, DNA, modified RNA, modified DNA, or combinations thereof.

In an embodiment of the third aspect prior, during or after step a) but before step c) the double-stranded molecule is separated into a first strand and a second strand.

In an embodiment of the third aspect in step e) it is detected whether the capture probe is hybridized to the first strand or the second strand.

In a fourth aspect the problem underlying the present invention is solved by a method for the detection in a sample of a nucleic acid molecule comprising at least a strand comprising a sequence of nucleotides, whereby the method comprises the following steps:
a) providing a sample containing the nucleic acid molecule;
b) providing a capture probe;
c) providing a bridging probe comprising a detection means, whereby a first part of the bridging probe is at least partially complementary to the capture probe, a second part of the bridging probe is at least partially complementary to said a strand of the nucleic acid molecule and a third part of the bridging probe is at least partially complementary to a detection probe, and whereby when the second part of the bridging probe is hybridized to said a strand of the nucleic acid, the third part of the bridging probe forms an overhang;
d) ligating one end of either the said a strand of the nucleic acid molecule to one end of the capture probe; and
detecting whether or not the bridging probe is hybridized to said a strand of the nucleic acid molecule ligated to the capture probe.

In an embodiment of the fourth aspect either the capture probe or said a strand of the nucleic acid molecule provides for the monophosphorylated 5' end required for the ligation in step d).

In an embodiment of the fourth aspect the capture probe and said a strand of the nucleic acid molecule, taken together, form a nucleotide sequence which is essentially complementary to a stretch of consecutive nucleotides comprising the second part of the bridging probe and at least a part of the first part of the bridging probe.

In an embodiment of the fourth aspect the capture probe provides for a monophosphorylated 5' end and the 3'end of said a strand of the nucleic acid molecule is ligated to said monophosphorylated 5' end of the capture probe, whereas a strand of the nucleic acid molecule the 3' end of which differs in the nucleotide sequence from the 3' end of said a strand of the nucleic acid molecule at the 3' end of one or more nucleotides will not be ligated.

In an embodiment of the fourth aspect the method further comprises the step of:
e) providing a detection probe, whereby the detection probe comprises a detection means which is different from the detection means of the bridging probe, and whereby the detection probe is at least partially complementary to the third part of the bridging probe;
f) ligating one end of said a strand of the nucleic acid to one end of the detection probe; and
g) detecting whether or not the detection probe is ligated to said a strand of the nucleic acid molecule.

In a preferred embodiment of the fourth aspect either the detection probe or said a strand of the nucleic acid molecule provides for the monophosphorylated 5' end required for the ligation in step g).

In an embodiment of the fourth aspect the detection probe and said a strand of the nucleic acid molecule, taken together, form a nucleotide sequence which is essentially complementary to a stretch of consecutive nucleotides comprising the second part of the bridging probe and at least a part of the third part of the bridging probe.

In an embodiment of the fourth aspect the detection probe provides for a 3' end and the 5'end of said a strand of the nucleic acid molecule provided for a monophosphorylated 5' end which is ligated to said 3' end of the detection probe, whereas a strand which differs from the 5' end of said a strand of the nucleic acid molecule at the 5' end of one or more nucleotides will not be ligated.

In an embodiment of the fourth aspect the detection means of the capture probe and the detection means of the detection probe form a FRET system or are at least a part thereof.

In an embodiment of the fourth aspect the length of the first part of the bridging probe is about 2 to 15 consecutive nucleotides, preferably 6 to 10 consecutive nucleotides.

In an embodiment of the fourth aspect the length of the second part of the bridging probe is essentially identical to the length of said a strand of the nucleic acid molecule.

In an embodiment of the fourth aspect the length of the third part of the bridging probe is about 2 to 20 consecutive nucleotides, preferably 10 to 15 nucleotides.

In an embodiment of the fourth aspect the bridging probe consist of a first molecule and a second molecule, whereby the first molecule is a single-stranded nucleic acid molecule comprising the first part of the bridging probe and the second part of the bridging probe, and the second molecule is a single-stranded nucleic acid molecule comprising the third part to the bridging probe.

In an embodiment of the fourth aspect the nucleic acid molecule is a single-stranded nucleic acid molecule.

In a preferred embodiment of the fourth aspect the single-stranded molecule consists of RNA, DNA, modified RNA or modified DNA, preferably DNA, RNA, modified RNA or modified DNA.

In an embodiment of the fourth aspect the nucleic acid molecule is a double-stranded nucleic acid comprising a first strand and a second strand, and wherein the strand of the nucleic acid molecule according to the fourth aspect of the present invention is the first strand or the second strand of the double-stranded nucleic acid, or a part thereof.

In a preferred embodiment of the fourth aspect the first strand or the second strand of the double-stranded nucleic acid consists each and independently from each other of RNA, DNA, modified RNA, modified DNA, or combinations thereof.

In an embodiment of the fourth aspect prior, during or after step a) but before step c) the double-stranded molecule is separated into a first strand and a second strand.

In an embodiment of the fourth aspect in step d) it is detected whether the capture probe is hybridized to the first strand or the second strand.

In a fifth aspect the problem underlying the present invention is solved by a method for the detection in a sample of a double-stranded nucleic acid molecule comprising a first strand and a second strand, whereby the method comprises the following steps:
a) providing a sample containing the double-stranded nucleic acid molecule;
b) providing a capture probe, whereby the capture probe is at least partially complementary to a part of either the first strand or the second strand of the double-stranded nucleic acid molecule and is immobilzable to a support,
c) providing a detection probe, whereby the detection probe is at least partially complementary to a part of either the second strand or the first strand of the double-stranded nucleic acid molecule and provides for a detection means;
d) allowing the sample and more specifically the double-stranded nucleic acid molecule contained therein, the capture probe and the detection probe to react; and
e) detecting whether the detection probe is attached to the double-stranded nucleic acid molecule and the capture probe is attached to the double-stranded nucleic acid molecule.

In an embodiment of the fifth aspect the attachement of step e) is a hybridization.

In an embodiment of the fifth aspect in step e) or d), the detection probe is attached to a part of the first strand of the double-stranded nucleic acid and the capture probe is attached to a part of the second strand of the double-stranded nucleic acid, or the detection probe is attached to a part of the second strand of the double-stranded nucleic acid and the capture probe is attached to a part of the first strand of the double-stranded nucleic acid.

In an embodiment of the fifth aspect the double-stranded nucleic acid molecule is present, at least in step d) and/or step e) as a double-stranded nucleic acid molecule.

In an embodiment of the fifth aspect the detection probe and the capture probe are attached to the same end of the double-stranded nucleic acid molecule, whereby such end is defined by the 5' end of one strand and the 3' end of the other strand of the double-stranded nucleic acid molecule. In an embodiment of the fifth aspect the detection probe and the capture probe are attached at different ends of the double-stranded nucleic acid molecule, whereby such ends are defined by the 5' end of the first strand and the 3' end of the second strand, and by the 3' end of the first strand and the 5' end of the second strand, respectively.

In an embodiment of the first to the fifth aspect the detection means is selected from the group comprising tags and labels.

In a preferred embodiment of the first to the fifth aspect the tag is selected from the group comprising biotin, streptavidin, avidin, nucleic acids, polypeptides and proteins.

In an embodiment of the first to the fifth aspect the tag is either directly or through a linker attached to a nucleotide, preferably the 5' terminal nucleotide.

In an embodiment of the first to the fifth aspect the label is selected from the group comprising radioactive labels, enzymatic labels, fluorescent labels, Cy-3, Cy-5, molecular beacons, bromo-desoxyuridine labels, a digoxigenin labels and chelator molecules.

In a preferred embodiment of the first to the fifth aspect the label is either directly or through a linker attached to a nucleotide, preferably the 5' terminal nucleotide.

In an embodiment of the first to the fifth aspect any of the probes is a single-stranded nucleic acid molecule.

In an embodiment of the first to the fifth aspect the nucleic acid molecule is a D-nucleic acid molecule or PNA based nucleic acid molecule and any of the capture probes, detection probes and bridging probes, each and independently, consists of nucleotides and derivatives, whereby such nucleotides and derivatives, respectively, are preferably selected from the group comprising D-deoxribonucleotides, modified D-deoxyribonucleotides, D-ribonucleotides, modified D-ribonucleotides, D-LNA nucleotides, PNA nucleotides, and any combination thereof.

In an embodiment of the first to the fifth aspect the nucleic acid molecule is a L-nucleic acid molecule and any of the capture probes, detection probes and bridging probes, each and independently, consists of nucleotides and derivatives, whereby such nucleotides and derivatives, respectively, are preferably selected from the group comprising L-deoxribonucleotides, modified L-deoxyribonucleotides, L-ribonucleotides, modified L-ribonucleotides, L-LNA nucleotides, and any combination thereof.

In an embodiment of the first to the fifth aspect the capture probe and/or any bridging probe is attached to the support either directly or through a linker.

In a preferred embodiment of the first to the fifth aspect the linker is selected from the group comprising hydrophilic linker, D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides L-LNA nucleotides, and any combination thereof.

As will be acknowledged by the one skilled in the art, the methods according to the first to the sixth aspect of the present invention are particularly suitable to detect single-stranded nucleic acids like aptamers and ribozymes or double-stranded nucleic acid molecules like RNA interference mediating molecules and decoy oligonucleotides, whereby the double-stranded nucleic acid molecules comprising a first strand and a second strand, whereby the first strand and the second strand are essentially complementary. As preferably used herein essentially complementary means that under the reaction conditions, a double-stranded structure is formed, whereby one or several nucleotide pairs are not base pairing or at least are not base pairing according to the established Watson-Crick base pair binding rules. It is also within the present invention that such double-stranded nucleic acid is formed by a single molecule, preferably by both strands being linked covalently to each other, by nucleotides or any other chemical entity.

It will also be acknowledged that, particularly in case of a method according to the second and third aspect of the present invention, also a nucleic acid molecule such as a spiegelmer, as defined herein in more detail, can be detected.

It will also be acknowledged that, particularly in case of a method according to the first, second and third aspect of the present invention, also a nucleic acid molecule such as a PNA molecule, as defined herein in more detail, can be detected.

It will also be acknowledged by the one skilled in the art that the various methods according to the present invention can be used for the detection as well as the quantification of the respective nucleic acid molecule. It will be further acknowledged that in any aspect of the present invention the nucleic acid is a specific nucleic acid molecule. As preferably used herein, a specific nucleic acid is a nucleic acid which binds solely to one mRNA sequence due to Watson-and-Crick-base-pairing or to one target whereby the binding to the target is not based on Watson-and-Crick-base-pairing.

It will also be acknowledged by the one skilled in the art that the order of the steps recited in the various methods are not necessarily reflecting the order according to which the various steps have to taken. Rather it will be obvious for the one skilled in the art that the order can be changed, whereby such changes are obvious for the one skilled in the art give the technical teaching provided herein. More specifically, in case the double-stranded nucleic acid molecule is, in a first step, provided as a double-stranded nucleic acid molecule, such double-stranded nucleic acid molecule can be treated such as to provide a first strand and a second strand. Methods are known to the one skilled in the art on how to separate such double-stranded nucleic acid molecule into said first and said second strand. Preferably such separation is performed by denaturation, for example by the heat and/or addition of salt to a reaction containing the double-stranded nucleic acid molecule. In case the individual strands are subject to further reactions, such strands may be separated, or can be used together, whereby, typically, the reaction conditions are such that the further reaction is subject to the various methods of the present invention can still be performed. Typically such measures comprise, but are not limited to, decreasing the temperature and/or increasing or decreasing the salt concentration and salt content, respectively.

The terms nucleic acid and nucleic acid molecule are used in an interchangeable manner herein if not indicated to the contrary.

It will be acknowledged by the ones skilled in the art that the nucleic acid molecule in accordance with the invention preferably consists of nucleotides which are covalently linked to each other, preferably through phosphodiester links or linkages. However, it will also be within the present invention that linkages other than phosphodiester may form the backbone of the nucleic acid molecules, including but not limited to phosphorothioates.

The term "part" of the nucleic acids shall mean as little as one nucleotide.

In a preferred embodiment each and any of the nucleic acid molecules described herein in their entirety in terms of their nucleic acid sequence(s) are limited to the particular nucleotide sequence(s). In other words, the terms "comprising" or "comprise(s)" shall be interpreted in such embodiment in the meaning of containing or consisting of.
L-nucleic acids as used herein are nucleic acids consisting of L-nucleotides, preferably consisting completely of L-nucleotides.
D-nucleic acids as used herein are nucleic acids consisting of D-nucleotides, preferably consisting completely of D-nucleotides.

Irrespective of whether the nucleic acid to be detected consists of D-nucleotides, L-nucleotides or a combination of both with the combination being e.g. a random combination or a defined sequence of stretches consisting of at least one L-nucleotide and at least one D-nucleic acid, the nucleic acid may consist of desoxyribonucleotide(s), ribonucleotide(s) or combinations thereof.

As used herein, a sample is preferably a sample which contains at least one nucleic acid molecule and the motivation for performing any of the methods according to the various aspects of the present invention is to determine whether a nucleic acid molecule is contained. Apart from any reaction in terms of a chemical reaction performed in a vessel to generate or characterise such nucleic acid molecule, the sample is preferably a sample taken from an organism to which such nucleic acid molecule has been administered. Accordingly, a sample may be a blood sample, a liquor sample, a faeces sample, urine sample, saliva sample, tears sample, lymph liquid sample or vaginal liquid sample.

It will also be acknowledged by the one skilled in the art that, when it is referred in the individual steps of the various methods according to the various aspects of the present invention it is referred to the use of either the first strand or the second strand, or a respective part thereof, of a double-stranded nucleic acid molecule, only one of these strands is subject to the respective step and reaction described therein, respectively.

It will also be acknowledged by the one skilled in the art that in a preferred embodiment, the capture probe is attached to a support, preferably a solid support such as, the surface of a reaction vessel or an array support.

More specifically with regard to the method according to the first aspect of the present invention, it will be acknowledged by the one skilled in the art that in this method, the read-out is based on the protection of the capture probe by the strand of the single-stranded nucleic acid or by one of either the first strand or the second strand of the double-stranded nucleic acid molecule binding thereto, against degradation.

The basic principle underlying the method according to the second aspect is based on the displacement of the detection probe by the strand of the single-stranded nucleic acid , e.g. aptamers, spiegelmers, ribozymes and antisense oligonucleotides, or by one of either the first or the second strand of the double-stranded nucleic acid molecule, e.g. RNA interference mediating molecules and decoy oligonucleotides, binding to the capture probe. Therefore, typically a reduction in the signal intensity is a measure for the presence, and the extent of the presence of such strand and, accordingly, ultimately the nucleic acid molecule.

The rational underlying the method according to the third and fourth aspect of the present invention is to ligate the strand of the single-stranded nucleic acid , e.g. aptamers, ribozymes and antisense oligonucleotides, or either one of the two strands of the double-stranded nucleic acid molecule, e.g. RNA interference mediating molecules and decoy oligonucleotides, to at least one or several of auxiliary nucleic acid molecules such as the capture probe and/or the detection probe. By doing so, the strand(s) of the nucleic acid molecule can be detected, more specifically, it can be assessed whether the respective strand(s) has/have the desired length. This can be realised by the ligation step which necessitates that the strand of the single-stranded nucleic acid or both the one end of either the first or the second strand of the double-stranded nucleic acid molecule is brought into close proximity of another end of a nucleic acid molecule such as either the capture probe or the detection probe, so as to allow the ligation reaction. In case at least one end or optionally also both ends of the nucleic acid molecule and more specifically the respective strand of the single-stranded nucleic acid or the first and second strand of the double-stranded nucleic acid, does not have the length as desired and as laid down in the bridging probe, such ligation will not occur. The extent of the ligation will then define under which conditions the lable attached to auxiliary nucleic acid molecules such as the detection probe can be separated from the single-stranded nucleic acid molecule or double-stranded nucleic acid molecule and either the first or the second strand thereof, respectively. Insofar, not only a qualitative analysis but also a quantitative analysis and more specifically a quantitative analysis discriminating between the desired single-stranded nucleic acid molecule or the double-stranded nucleic acid molecule and its first and second strand, respectively, is possible but the discrimination between the desired molecule and possible degradation products thereof.

The method according to the sixth aspect of the present invention is particularly advantageous insofar that here actually a double-stranded nucleic acid molecule is detected as such, i. e. in contrast to the other methods where the constituents thereof, namely the first strand and the second strand forming the double-stranded nucleic acid molecule are individually detected.

It will be acknowledged by the one skilled in the art that factually any nucleic acid molecule and more specifically any double-stranded nucleic acid molecule can be detected using the method as described herein. A particularly preferred species of molecules are RNA interference mediating molecules which are also referred to herein as siRNA molecules.

The basic design of siRNA molecules, miRNA molecules or RNAi molecules, which mostly differ in the size, is basically such that the nucleic acid molecule comprises a double-stranded structure. The double-stranded structure comprises a first strand and a second strand. More preferably, the first strand comprises a first stretch of contiguous nucleotides and the second stretch comprises a second stretch of contiguous nucleotides. At least the first stretch and the second stretch are essentially complementary to each other. Such complementarity is typically based on Watson-Crick base pairing or other base-pairing mechanism known to the one skilled in the art, including but not limited to Hoogsteen base-pairing and others. It will be acknowledged by the one skilled in the art that depending on the length of such double-stranded structure a perfect match in terms of base complementarity is not necessarily required. However, such perfect complementarity is preferred in some embodiments. A mismatch is also tolerable, mostly under the proviso that the double-stranded structure is still suitable to trigger the RNA interference mechanism, and that preferably such double-stranded structure is still stably forming under physiological conditions as prevailing in a cell, tissue and organism, respectively, containing or in principle containing such cell, tissue and organ. More preferably, the double-stranded structure is stable at 37 °C in a physiological buffer.

The first stretch, is typically at least partially complementary to a target nucleic acid and the second stretch is, particularly given the relationship between the first and second stretch, respectively, in terms of base complementarity, at least partially identical to the target nucleic acid. The target nucleic acid is preferably an mRNA, although other forms of RNA such as hnRNAs are also suitable for such purpose. Such siRNA molecule, miRNA molecule and RNAi molecule respectively, is suitable to trigger the RNA interference response resulting in the knock-down of the mRNA for the target molecule. Insofar, this kind of nucleic acid molecule is suitable to decrease the expression of a target molecule by decreasing the expression at the level of mRNA.

Although RNA interference can be observed upon using long nucleic acid molecules comprising several dozens and sometimes even several hundreds of nucleotides and nucleotide pairs, respectively, shorter siRNA molecules, miRNA molecules and RNAi molecules are generally preferred. A more preferred range for the length of the first stretch and/or second stretch is from about 15 to 29 consecutive nucleotides, preferably 19 to 25 consecutive nucleotides and more preferably 19 to 23 consecutive nucleotides. More preferably, both the first stretch and the second stretch have the same length. In a further embodiment, the double-stranded structure comprises preferably between 15 and 29, preferably 18 to 25, more preferably 19 to 23 and most preferably 19 to 21 base pairs.

It will be acknowledged by the ones skilled in the art that the particular design of the siRNA molecules, miRNA molecules, the RNAi molecules and other nucleic acids mediating RNAi, respectively, can vary in accordance with the current and future design principles. For the time being some design principles of the siRNA molecules, miRNA molecules and the RNAi molecules and other nucleic acids mediating RNAi, respectively, exist which shall be discussed in more detail in the following and referred to as sub-aspects the present invention which is related to the detection of siRNA molecules, miRNA molecules, the RNAi molecules and other nucleic acids mediating RNAi, respectively.

In the following siRNA molecules, miRNA molecules and the RNAi molecules and other nucleic acids mediating RNAi are jointly called 'small interfering nucleic acid' (siNA) molecules.

The nucleic acid to be analysed according to the present invention preferably shares all functional features are known for siRNA, as previously described herein.

As described before for siRNA molecules, miRNA molecules or RNAi molecules the siNA subject to the method according to the present invention comprises a double-stranded structure. The double-stranded structure comprises a first strand and a second strand. More preferably, the first strand comprises a first stretch of contiguous nucleotides and the second stretch comprises a second stretch of contiguous nucleotides. At least the first stretch and the second stretch are essentially complementary to each other. Such complementarity is typically based on Watson-Crick base pairing or other base-pairing mechanism known to the one skilled in the art, including but not limited to Hoogsteen base-pairing and others. The first stretch, is typically at least partially complementary to a target nucleic acid and the second stretch is, particularly given the relationship between the first and second stretch, respectively, in terms of base complementarity, at least partially identical to the target nucleic acid. In a preferred embodiment of the siNA according to the present invention complementarity between said first strand and the target nucleic acid is perfect.

In a first subaspect the first stretch and/or the second stretch of the siNA subject to the methods according to the present invention comprise/s modified nucleotides having a modification at the 2' position and/or non-modified nucleotides, so that the first stretch and/or the second stretch of the siNA subject to the methods according to the present invention fully consists of modified or unmodified nucleotides or a mixture of modified and un-modified nucleotides. The percentage rate of the modified within all nucleotides of each stretch can independently be 0 -100%.

In a preferred embodiment, the first stretch of the siNA according to the present invention comprises a plurality of group of modified nucleotides having a modification at the 2' position, whereby within the stretch each group of modified nucleotides is flanked on one or both sides by a flanking group of nucleotides, whereby the flanking nucleotides forming the flanking group of nucleotides is either an unmodified nucleotide or a nucleotide having a modification different from the modification of the modified nucleotides. Such design is, among others described in international patent application WO 2004/015107. The the siNA nucleic acid according to this aspect is preferably a ribonucleic acid although, as will be outlined in some embodiments, the modification at the 2' position results in a nucleotide which as such is, from a pure chemical point of view, no longer a ribonucleotide. However, it is within the present invention that such modified ribonucleotide shall be regarded and addressed herein as a ribonucleotide and the molecule containing such modified ribonucleotide as a ribonucleic acid.

In an embodiment of the siNA subject to the method according to the present invention is blunt ended, either on one side or on both sides of the double-stranded structure. In a more preferred embodiment the double-stranded structure comprises 18 or 19 base pairs. In an even more preferred embodiment, the siNA subject to the methods according to the present invention consists of the first stretch and the second stretch only.

In another embodiment of the siNA subject to the method according to the present invention at least one of the two strands has an overhang of at least one nucleotide at the 5'-end.

In a preferred embodiment of the the siNA subject to the method according to the present invention the overhang consists of at least one deoxyribonucleotide.

In a further embodiment of the siNA subject to the method according to the present invention at least one of the strands has an overhang of at least one nucleotide at the 3'-end.

In a further embodiment of the siNA subject to the method according to the present invention said first stretch and/or said second stretch comprise a plurality of groups of modified nucleotides. In a further preferred embodiment the first stretch and/or the second stretch also comprises a plurality of flanking groups of nucleotides. In a preferred embodiment a plurality of groups means at least two groups.

In a further preferred embodiment both the first and the second stretch comprise a plurality of both groups of modified nucleotides and flanking groups of nucleotides. In a more preferred embodiment the plurality of both groups of modified nucleotides and flanking groups of nucleotides form a pattern, preferably a regular pattern, on either the first stretch and/or the second stretch, whereby it is even more preferred that such pattern is formed on both the first and the second stretch.

In another embodiment of the siNA subject to the method according to the present invention the pattern of modified nucleotides of said first stretch is the same as the pattern of modified nucleotides of said second stretch.

In a preferred embodiment of the siNA subject to the method according to the present invention the pattern of said first stretch aligns with the pattern of said second stretch.

In an alternative embodiment of the siNA subject to the method according to the present invention the pattern of said first stretch is shifted by one or more nucleotides relative to the pattern of the second stretch.

In an embodiment of the ribonucleic acid according to the first sub-aspect of the present invention the modification at the 2' position is selected from the group comprising amino, fluoro, methoxy, alkoxy and alkyl.

In another embodiment of the siRNA subject to the method according to the present invention the double stranded structure is blunt ended.

In an embodiment of the ribonucleic acid according to the first sub-aspect both the first strand and the second strand each comprise at least one group of modified nucleotides and at least one flanking group of nucleotides, whereby each group of modified nucleotides comprises at least one nucleotide and whereby each flanking group of nucleotides comprising at least one nucleotide with each group of modified nucleotides of the first strand being aligned with a flanking group of nucleotides on the second strand, whereby the most terminal 5' nucleotide of the first strand is a nucleotide of the group of modified nucleotides, and the most terminal 3' nucleotide of the second strand is a nucleotide of the flanking group of nucleotides.

In a preferred embodiment of the ribonucleic acid according to of the first sub-aspect, each group of modified nucleotides consists of a single nucleotide and/or each flanking group of nucleotides consists of a single nucleotide.

In a further embodiment of the ribonucleic acid according to the first sub-aspect, on the first strand the nucleotide forming the flanking group of nucleotides is an unmodified nucleotide which is arranged in a 3' direction relative to the nucleotide forming the group of modified nucleotides, and wherein on the second strand the nucleotide forming the group of modified nucleotides is a modified nucleotide which is arranged in 5' direction relative to the nucleotide forming the flanking group of nucleotides.

The ribonucleic acid molecule according to such first sub-aspect may be designed is to have a free 5' hydroxyl group, also referred to herein as free 5' OH-group, at the first strand. A free 5' OH-group means that the most terminal nucleotide forming the first strand is present and is thus not modified, particularly not by an end modification. Typically, the terminal 5 '-hydroxy group of the second strand, respectively, is also present in an unmodified manner. In a more preferred embodiment, also the 3'-end of the first strand and first stretch, respectively, is unmodified such as to present a free OH-group which is also referred to herein as free 3'OH-group, whereby the design of the 5' terminal nucleotide is the one of any of the afore-described embodiments. Preferably such free OH-group is also present at the 3'-end of the second strand and second stretch, respectively. In other embodiments of the ribonucleic acid molecules as described previously according to the present invention the 3'-end of the first strand and first stretch, respectively, and/or the 3'-end of the second strand and second stretch, respectively, may have an end modification at the 3' end.

As used herein the terms free 5'OH-group and 3'OH-group also indicate that the respective most terminal nucleotide at the 5'end and the 3' end of the polynucleotide, respectively, i.e. either the nucleic acid or the strands and stretches, respectively, forming the double-stranded structure present an OH-group. Such OH-group may stem from either the sugar moiety of the nucleotide, more preferably from the 5'position in case of the 5'OH-group and from the 3'position in case of the 3'OH-group, or from a phosphate group attached to the sugar moiety of the respective terminal nucleotide. The phosphate group may in principle be attached to any OH-group of the sugar moiety of the nucleotide. Preferably, the phosphate group is attached to the 5'OH-group of the sugar moiety in case of the free 5'OH-group and/or to the 3'OH-group of the sugar moiety in case of the free 3'OH-group still providing what is referred to herein as free 5' or 3' OH-group.

As used herein with any embodiment of the first sub-aspect, the term end modification means a chemical entity added to the most 5' or 3' nucleotide of the first and/or second strand. Examples for such end modifications include, but are not limited to, inverted (deoxy) abasics, amino, fluoro, chloro, bromo, CN, CF, methoxy, imidazole, caboxylate, thioate, C₁ to C₁₀ lower alkyl, substituted lower alkyl, alkaryl or aralkyl, OCF₃, OCN, O-, S-, or N-alkyl; O-, S-, or N-alkenyl; SOCH₃; SO₂CH₃; ONO₂; NO₂, N₃; heterozycloalkyl; heterozycloalkaryl; aminoalkylamino; polyalkylamino or substituted silyl, as, among others, described in European patents EP 0 586 520 B1 or EP 0 618 925 B1.

As used herein, alkyl or any term comprising "alkyl" means any carbon atom chain comprising 1 to 12, preferably 1 to 6 and more, preferably 1 to 2 C atoms.

A further end modification is a biotin group. Such biotin group may preferably be attached to either the most 5' or the most 3' nucleotide of the first and/or second strand or to both ends. In a more preferred embodiment the biotin group is coupled to a polypeptide or a protein. It is also within the scope of the present invention that the polypeptide or protein is attached through any of the other aforementioned end modifications. The polypeptide or protein may confer further characteristics to the inventive nucleic acid molecules. Among others the polypeptide or protein may act as a ligand to another molecule. If said other molecule is a receptor the receptor's function and activity may be activated by the binding ligand. The receptor may show an internalization activity which allows an effective transfection of the ligand bound inventive nucleic acid molecules. An example for the ligand to be coupled to the inventive nucleic acid molecule is VEGF and the corresponding receptor is the VEGF receptor.

Various possible embodiments of the RNAi of the present invention having different kinds of end modification(s) are presented in the following table 1.

**Table 1: Various embodiments of the interfering ribonucleic acid according to the present invention**

| | | **1^{st} strand/1^{st} stretch** | **2^{nd} strand/ 2nd stretch** |
|---|---|---|---|
| **1.)** | **5'-end** | free OH | free OH |
| | **3'-end** | free OH | free OH |
| | | | |
| **2.)** | **5'-end** | free OH | free OH |
| | **3'-end** | end modification | end modification |
| | | | |
| **3.)** | **5'-end** | free OH | free OH |
| | **3'-end** | free OH | end modification |
| | | | |
| **4.)** | **5'-end** | free OH | free OH |
| | **3'-end** | end modification | free OH |
| | | | |
| **5.)** | **5'-end** | free OH | end modification |
| | **3'-end** | free OH | free OH |
| | | | |
| **6.)** | **5'-end 3'-end** | free OH end modification | end modification free OH |
| | | | |
| **7.)** | **5'-end** | free OH | end modification |
| | **3'-end** | free OH | end modification |
| | | | |
| **8.)** | **5'-end** | free OH | end modification |
| | **3'-end** | end modification | end modification |

The various end modifications as disclosed herein are preferably located at the ribose moiety of a nucleotide of the ribonucleic acid. More particularly, the end modification may be attached to or replace any of the OH-groups of the ribose moiety, including but not limited to the 2'OH, 3'OH and 5'OH position, provided that the nucleotide thus modified is a terminal nucleotide. Inverted abasics are nucleotides, either desoxyribonucleotides or ribonucleotides which do not have a nucleobase moiety. This kind of compound is, among others, described in Sternberger et al.(2002), Antisense. Nucl. Ac. Drug Dev. 12(3):131-43.

Any of the aforementioned end modifications may be used in connection with the various embodiments of RNAi depicted in table 1. In connection therewith it is to be noted that any of the RNAi forms or embodiments disclosed herein with the sense strand being inactivated, preferably by having an end modification more preferably at the 5' end, are particularly advantageous. This arises from the inactivation of the sense strand which corresponds to the second strand of the ribonucleic acids described herein, which might otherwise interfere with an unrelated single-stranded RNA in the cell. Thus the expression and more particularly the translation pattern of the transcriptome of a cell is more specifically influenced. This effect is also referred to as off-target effect.

In a further embodiment, the nucleic acid according to the first sub-aspect has an overhang at the 5'-end of the ribonucleic acid. More particularly, such overhang may in principle be present at either or both the first strand and second strand of the ribonucleic acid according to the present invention. The length of said overhang may be as little as one nucleotide and as long as 2 to 8 nucleotides, preferably 2, 4, 6 or 8 nucleotides. It is within the present invention that the 5' overhang may be located on the first strand and/or the second strand of the ribonucleic acid according to the present application. The nucleotide(s) forming the overhang may be (a) desoxyribonucleotide(s), (a) ribonucleotide(s) or a continuation thereof.

The overhang preferably comprises at least one desoxyribonucleotide, whereby said one desoxyribonucleotide is preferably the most 5'-terminal one.

Taken the stretch of contiguous nucleotides a pattern of modification of the nucleotides forming the stretch may be realised such that a single nucleotide or group of nucleotides which are covalently linked to each other via standard phosphorodiester bonds or, at least partially, through phosphorothioate bonds, show such kind of modification. In case such nucleotide or group of nucleotides which is also referred to herein as group of modified nucleotides, is not forming the 5'-end or 3'-end of said stretch a nucleotide or group of nucleotides follows on both sides of the nucleotide which does not have the modification of the preceding nucleotide or group of nucleotides. It is to be noted that this kind of nucleotide or group of nucleotides, however, may have a different modification. This kind of nucleotide or group of nucleotides is also referred to herein as the flanking group of nucleotides. This sequence of modified nucleotide and group of modified nucleotides, respectively, and unmodified or differently modified nucleotide or group of unmodified or differently modified nucleotides may be repeated one or several times. Preferably, the sequence is repeated more than one time. For reason of clarity the pattern is discussed in more detail in the following, generally referring to a group of modified nucleotides or a group of unmodified nucleotides whereby each of said group may actually comprise as little as a single nucleotide. Unmodified nucleotide as used herein means either not having any of the afore-mentioned modifications at the nucleotide forming the respective nucleotide or group of nucleotides, or having a modification which is different from the one of the modified nucleotide and group of nucleotides, respectively.

It is also within the present invention that the modification of the unmodified nucleotide(s) wherein such unmodified nucleotide(s) is/are actually modified in a way different from the modification of the modified nucleotide(s), can be the same or even different for the various nucleotides forming said unmodified nucleotides or for the various flanking groups of nucleotides.

The pattern of modified and unmodified nucleotides may be such that the 5'-terminal nucleotide of the strand or of the stretch starts with a modified group of nucleotides or starts with an unmodified group of nucleotides. However, in an alternative embodiment it is also possible that the 5'-terminal nucleotide is formed by an unmodified group of nucleotides.

This kind of pattern may be realised either on the first stretch or the second stretch of the interfering RNA or on both. It has to be noted that a 5' phosphate on the target-complementary strand of the siRNA duplex is required for siRNA function, suggesting that cells check the authenticity of siRNAs through a free 5' OH (which can be phosphorylated) and allow only such bona fide siRNAs to direct target RNA destruction (Nykanen, et al. (2001), Cell 107, 309-21).

Preferably, the first stretch shows a kind of pattern of modified and unmodified groups of nucleotides, i. e. of group(s) of modified nucleotides and flanking group(s) of nucleotides, whereas the second stretch does not show this kind of pattern. This may be useful insofar as the first stretch is actually the more important one for the target-specific degradation process underlying the interference phenomenon of RNA so that for specificity reasons the second stretch can be chemically modified so it is not functional in mediating RNA interference.

However, it is also within the present invention that both the first stretch and the second stretch have this kind of pattern. Preferably, the pattern of modification and non-modification is the same for both the first stretch and the second stretch.

In a preferred embodiment the group of nucleotides forming the second stretch and corresponding to the modified group of nucleotides of the first stretch are also modified whereas the unmodified group of nucleotides of or forming the second stretch correspond to the unmodified group of nucleotides of or forming the first stretch. Another alternative is that there is a phase shift of the pattern of modification of the first stretch and first strand, respectively, relative to the pattern of modification of the second stretch and second strand, respectively. Preferably, the shift is such that the modified group of nucleotides of the first strand corresponds to the unmodified group of nucleotides of the second strand and vice versa. It is also within the present invention that the phase shift of the pattern of modification is not complete but overlapping.

In a preferred embodiment the second nucleotide at the terminus of the strand and stretch, respectively, is an unmodified nucleotide or the beginning of group of unmodified nucleotides. Preferably, this unmodified nucleotide or unmodified group of nucleotides is located at the 5'- end of the first and second strand, respectively, and even more preferably of the first strand. In a further preferred embodiment the unmodified nucleotide or unmodified group of nucleotide is located at the 5'-end of the first strand and first stretch, respectively. In a preferred embodiment the pattern consists of alternating single modified and unmodified nucleotides.

In a further preferred embodiment of this aspect of the present invention the interfering ribonucleic acid subject comprises two strands, whereby a 2'-O-methyl modified nucleotide and a non-modified nucleotide, preferably a nucleotide which is not 2'-O-methyl modified, are incorporated on both strands in an alternate manner which means that every second nucleotide is a 2'-O-methyl modified and a non-modified nucleotide, respectively. This means that on the first strand one 2'-O-methyl modified nucleotide is followed by a non-modified nucleotide which in turn is followed by 2'-O-methyl modified nucleotide and so on. The same sequence of 2'-O-methyl modification and non-modification exists on the second strand, whereby there is preferably a phase shift such that the 2'-O-methyl modified nucleotide on the first strand base pairs with a non-modified nucleotide(s) on the second strand and vice versa. This particular arrangement, i. e. base pairing of 2'-O-methyl modified and non-modified nucleotide(s) on both strands is particularly preferred in case of short interfering ribonucleic acids, i. e. short base paired double-stranded ribonucleic acids because it is assumed, although the present inventors do not wish to be bound by that theory, that a certain repulsion exists between two base-pairing 2'-O-methyl modified nucleotides which would destabilise such duplex, preferably short duplexes. About the particular arrangement, it is preferred if the antisense strand starts with a 2'-O-methyl modified nucleotide at the 5' end whereby consequently the second nucleotide is non-modified, the third, fifth, seventh and so on nucleotides are thus again 2'-O-methyl modified whereas the second, fourth, sixth, eighth and the like nucleotides are non-modified nucleotides. Again, not wishing to be bound by any theory, it seems that a particular importance may be ascribed to the second, and optionally fourth, sixth, eighth and/or similar position(s) at the 5' terminal end of the antisense strand which should not comprise any modification, whereas the most 5' terminal nucleotide, i. e. the first 5' terminal nucleotide of the antisense strand may exhibit such modification with any uneven positions such as first, optionally third, fifth and similar position(s) at the antisense strand may be modified. In further embodiments the modification and non-modification, respectively, of the modified and non-modified nucleotide(s), respectively, may be anyone as described herein.

It is within the present invention that the double-stranded structure is formed by two separate strands, i.e. the first and the second strand. However, it is also with in the present invention that the first strand and the second strand are covalently linked to each other. Such linkage may occur between any of the nucleotides forming the first strand and second strand, respectively. However, it is preferred that the linkage between both strands is made closer to one or both ends of the double-stranded structure. Such linkage can be formed by covalent or non-covalent linkages. Covalent linkage may be formed by linking both strands one or several times and at several positions, respectively, by a compound selected from the group comprising methylene blue and bifunctinoal groups. Such bifunctional groups are preferably selected from the group comprising bis(2-chloroethyl)amine, N-acetly-N'-(p-glyoxylbenzoyl)cystamine, 4.thiouracil and psoralene.

In a further embodiment of the ribonucleic acid according to any of the first sub-aspects of the present invention the first strand and the second strand are linked by a loop structure.

In a preferred embodiment of the ribonucleic acid according to the first sub-aspects of the present invention the loop structure is comprised of a non-nucleic acid polymer.

In a preferred embodiment thereof the non-nucleic acid polymer is polyethylene glycol.

In an embodiment of the ribonucleic acid according to any of the first sub-aspects of the present invention the 5'-terminus of the first strand is linked to the 3'-terminus of the second strand.

In a further embodiment of the ribonucleic acid according to any of the aspects of the present invention the 3'-end of the first strand is linked to the 5'-terminus of the second strand.

In an embodiment the loop consists of a nucleic acid. As used herein, LNA as described in Elayadi and Corey (2001) Curr Opin Investig Drugs. 2(4):558-61. Review; Orum and Wengel (2001) Curr Opin Mol Ther. 3(3):239-43; and PNA are regarded as nucleic acids and may also be used as loop forming polymers. Basically, the 5'-terminus of the first strand may be linked to the 3'-terminus of the second strand. As an alternative, the 3'-end of the first strand may be linked to the 5'-terminus of the second strand. The nucleotide sequence forming said loop structure is regarded as in general not being critical. However, the length of the nucleotide sequence forming such loop seems to be critical for sterical reasons. Accordingly, a minimum length of four nucleotides seems to be appropriate to form the required loop structure. In principle, the maximum number of nucleotides forming the hinge or the link between both stretches to be hybridized is not limited. However, the longer a polynucleotide is, the more likely secondary and tertiary structures are formed and thus the required orientation of the stretches affected. Preferably, a maximum number of nucleotides forming the hinge is about 12 nucleotides. It is within the disclosure of this application that any of the designs described above may be combined with the afore-described sixth strategy, i. e. by linking the two strands covalently in a manner that back folding (loop) can occur through a loop structure or similar structure.

Insofar a preferred arrangement in 5' → 3' direction of this kind of small interfering RNAi is second strand - loop - first strand, whereby first stretch and/or the second stretch comprise at the 3' end a dinucleotide, whereby such dinucleotide is preferably TT. The design of the nucleic acid in accordance with this sub-aspect is described in more detail in e.g., in international patent application WO 01/75164.

The third sub-aspect of the first aspect of the present invention is related to a nucleic acid according to the present invention, whereby the first and/or the second stretch comprise an overhang of 1 to 5 nucleotides at the 3' end. The design of the nucleic acid in accordance with this sub-aspect is described in more detail in international patent application WO02/44321. More preferably such overhang is a ribonucleic acid.

In one embodiment thereof the siNA molecule comprises no ribonucleotides. In another embodiment, the siNA molecule comprises one or more nucleotides. In another embodiment chemically modified nucleotide comprises a 2'-deoxy nucleotide. In another embodiment chemically modified nucleotide comprises a 2'-deoxy-2'-fluoro nucleotide. In another embodiment chemically modified nucleotide comprises a 2'-O-methyl nucleotide. In another embodiment chemically modified nucleotide comprises a phosphorothioate internucleotide linkage. In a further embodiment the non-nucleotide comprises an abasic moiety, whereby preferably the abasic moiety comprises an inverted deoxyabasic moiety. In another embodiment non-nucleotide comprises a glyceryl moiety.

In a further embodiment of the nucleic acid according to the fifth sub-aspect, the pyrimidine nucleotides in the second strand are 2'-O-methyl pyrimidine nucleotides.

In a further embodiment of the nucleic acid according to the fifth sub-aspect, the purine nucleotides in the second strand are 2'-deoxy purine nucleotides.

In a further embodiment of the nucleic acid according to the fifth sub-aspect, the pyrimidine nucleotides in the second strand are 2'-deoxy-2'-fluoro pyrimidine nucleotides.

In a further embodiment of the nucleic acid according to the fifth sub-aspect, the second strand includes a terminal cap moiety at the 5' end, the 3' end or both the 5' end and the 3' end.

In a further embodiment of the nucleic acid according to the fifth sub-aspect, the pyrimidine nucleotides in the first strand are 2'-deoxy-2' fluoro pyrimidine nucleotides.

In a further embodiment of the nucleic acid according to the fifth sub-aspect, the purine nucleotides in the first strand are 2'-O-methyl purine nucleotides.

In a further embodiment of the nucleic acid according to the fifth sub-aspect, the purine nucleotides in the first strand are 2'-deoxy purine nucleotides.

In a further embodiment of the nucleic acid according to the fifth sub-aspect, the first strand comprises a phosphorothioate internucleotide linkage at the 3' end of the first strand.

In a further embodiment of the nucleic acid according to the fifth sub-aspect, the first strand comprises a glyceryl modification ar the 3' end of the first strand.

In a further embodiment of the nucleic acid according to the fifth sub-aspect, about 19 nucleotides of both the first and the second strand are base-paired and wherein preferably at least two 3' terminal nucleotides of each strand of the siNA molecule are not base-paired to the nucleotides of the other strand. Preferably, each of the two 3' terminal nucleotides of each strand of the siNA molecule are 2'-deoxy-pyrimidines. More preferably, the 2'deoxy-pyrimidine is 2' deoxy-thymidine.

In a further aspect of the nucleic acid according to the fifth sub-aspect, the 5' end of the first strand comprises a phosphate group.

In one embodiment particularly of the fifth sub-aspect of the nucleic acid according to the present invention, a siNA molecule of the invention comprises modified nucleotides while maintaining the ability to mediate RNAi. The modified nucleotides can be used to improve in *vitro* or *in vivo* characteristics such as stability, activity, and/or bioavailability. For example, a siNA molecule of the invention can comprise modified nucleotides as a percentage of the total number of nucleotides present in the siNA molecule. As such, a siNA molecule of the invention can generally comprise about 5 % to about 100 % modified nucleotides (e. g., 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, 95 % or 100 % modified nucleotides). The actual percentage of modified nucleotides present in a given siNA molecule will depend on the total number of nucleotides present in the siNA. If the siNA molecule is single stranded, the percent modification can be based upon the total number of nucleotides present in the single stranded siNA molecules. Likewise, if the siNA molecule is double stranded, the percent modification can be based upon the total number of nucleotides present in the sense strand, antisense strand, or both the sense and antisense strands.

The 3'-terminal nucleotide overhangs of a siNA molecule of the invention can comprise ribonucleotides or deoxyribonucleotides that are chemically-modified at a nucleic acid sugar, base or backbone. The 3'-terminal nucleotide overhangs can comprise one or more universal base ribonucleotides. The 3'-terminal nucleotide overhangs can comprise one or more acyclic nucleotides.

It will be acknowledged that what has been described herein as subaspects and embodiments, respectively, of the siRNA or RNA according to the present invention is meant to refer to the nucleic acid molecule and in particular the double-stranded nucleic acid molecule subject to the methods and kit, respectively, of the present invention.

The nucleic acid molecules of the present invention can be modified extensively to enhance stability by modification with nuclease resistant groups, for example, 2'-amino, 2'-C-allyl, 2'-fluoro, 2'-O-methyl, 2'-H (for a review see Usman and Cedergren, 1992, TIBS 17, 34; Usman et al., 1994, Nucleic Acids Symp. Ser. 31, 163). The nucleic acid molecules can be purified by gel electrophoresis using general methods or cn be purified by high pressure liquid chromatography (HPLC; see Wincott et al., 1995, Nucleic Acids Res. 23, 2677; Caruthers et al., 19922, Methods in Enzymology 211,3-19 (incorporated by reference herein)) and re-suspended in water.

As already described above, chemically synthesizing nucleic acid molecules with modifications (base, sugar and/or phosphate) can prevent their degradation by serum ribonucleases, which can increase their potency (see, e.g., Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; Usman et al., International Publication No. WO 91/03162; Sproat, U.S. Pat. No. 5,334,711; Gold et al., U.S. Pat. No. 6,300,074; and Burgin et al., supra; all of which are incorporated by reference herein). All of the above references describe various chemical modifications that can be made to the base, phosphate and/or sugar moieties of the nucleic acid molecules described herein. Modifications that enhance their efficacy in cells, and removal of bases from nucleic acid molecules to shorten oligonucleotide synthesis times and reduce chemical requirements are desired.

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-C-allyl, 2'-fluoro, 2'-O-methyl, 2'-O-allyl, 2'-H, nucleotide base modifications (for a review see Usman and Cedergren, 1992, TIBS. 17, 34; Usman et al., 1994, Nucleic Acids Symp. Ser. 31, 163; Burgin et al., 1996, Biochemistry, 35, 14090). Sugar modification of nucleic acid molecules have been extensively described in the art (see Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci., 1992, 17, 334-339; Usman et al. International Publication PCT No. WO 93/15187; Sproat, U.S. Pat. No. 5,334,711 and Beigelman et al., 1995, J. Biol. Chem., 270, 25702; Beigelman et al., International PCT publication No. WO 97/26270; Beigelman et al., U.S. Pat. No. 5,716,824; Usman et al., U.S. Pat. No. 5,627,053; Woolf et al., International PCT Publication No. WO 98/13526; Thompson et al., USSN 60/082,404 which was filed on April 20, 1998; Karpeisky et al., 1998, Tetrahedron Lett., 39, 1131; Earnshaw and Gait, 1998, Biopolymers (Nucleic Acid Sciences), 48, 39-55; Verma and Eckstein, 1998, Annu. Rev. Biochem., 67, 99-134; and Burlina et al., 1997, Bioorg. Med. Chem., 5, 1999-2010; all of the references are hereby incorporated in their totality by reference herein). Such publications describe general methods and strategies to determine the location of incorporation of sugar, base and/or phosphate modifications and the like into nucleic acid molecules without modulating catalysis, and are incorporated by reference herein. In view of such teachings, similar modifications can be used as described herein to modify the nucleic acid molecules of the instant invention so long as the ability of such nucleic acid molecules to promote RNAi in cells is not significantly inhibited.

While chemical modification of oligonucleotide internucleotide linkages with phosphorothioate, phosphorodithioate, and/or 5'-methylphosphonate linkages improves stability, excessive modifications can cause some toxicity or decreased activity. Therefore, when designing nucleic acid molecules, the amount of these internucleotide linkages should be minimized. The reduction in the concentration of these linkages should lower toxicity, resulting in increased efficacy and higher specificity of these molecules.

Short interfering nucleic acid molecules which are an embodiment of the nucleic acid molecules according to the present invention and which are also referred to as siNA molecules, having chemical modification that maintain or enhance activity are provided. Such a nucleic acid is also generally more resistant to nucleases than an unmodified nucleic acid. Accordingly, the in vitro and/or in vivo activity should not be significantly lowered. In cases in which modulation is the goal, therapeutic nucleic acid molecules delivered exogenously should optimally be stable within cells until translation of the target RNA has been modulated long enough to reduce the levels of the undesirable protein. This period of time varies between hours to days depending upon the disease state. Improvements in the chemical synthesis of RNA and DNA (Wincott et al., 1995, Nucleic Acids Res. 23, 2677; Caruthers et al., 19922, Methods in Enzymology 211,3-19 (incorporated by reference herein)) have expanded the ability to modify nucleic acid molecules by introducing nucleotide modifications to enhance their nuclease stability, as described above.

In one embodiment, nucleic acid molecules of the invention include one or more (e.g., about 1, 2, 3, 4, 5, 6, 7, 8, 9, 10, or more) G-clamp nucleotides. A G-clamp nucleotide is a modified cytosine analog wherein the modifications confer the ability to hydrogen bond both Watson-Crick and Hoogsteen faces of a complementary guanine within a duplex, see for example Lin and Matteucci, 1998, J. Am. Chem. Soc., 120, 8531-8532. A single G-clamp analog substitution within an oligonucleotide can result in substantially enhanced helical thermal stability and mismatch discrimination when hybridized to complementary oligonucleotides. The inclusion of such nucleotides in nucleic acid molecules of the invention results in both enhanced affinity and specificity to nucleic acid targets, complementary sequences, or template strands. In another embodiment, nucleic acid molecules of the invention include one or more (e.g., about 1, 2, 3, 4, 5, 6, 7, 8, 9, 10, or more) LNA "locked nucleic acid" nucleotides such as a 2', 4'-C methylene bicycle nucleotide (see for example Wengel et al., International PCT Publication No. WO 00/66604 and WO 99/14226).

In another embodiment, the invention features conjugates and/or complexes of siNA molecules of the invention. Such conjugates and/or complexes can be used to facilitate delivery of nucleic acid molecules into a biological system, such as a cell. The conjugates and complexes provided by the instant invention can impart therapeutic activity by transferring therapeutic compounds across cellular membranes, altering the pharmacokinetics, and/or modulating the localization of nucleic acid molecules of the invention. The present invention encompasses the design and synthesis of novel conjugates and complexes for the delivery of molecules, including, but not limited to, small molecules, lipids, phospholipids, nucleosides, nucleotides, nucleic acids, antibodies, toxins, negatively charged polymers and other polymers, for example proteins, peptides, hormones, carbohydrates, polyethylene glycols, or polyamines, across cellular membranes. In general, the transporters described are designed to be used either individually or as part of a multi-component system, with or without degradable linkers. These compounds are expected to improve delivery and/or localization of nucleic acid molecules of the invention into a number of cell types originating from different tissues, in the presence or absence of serum (see Sullenger and Cech, u.S. Pat. No. 5,854,038). Conjugates of the molecules described herein can be attached to biologically active molecules via linkers that are biodegradable, such as biodegradable nucleic acid linker molecules.

The term "biodegradable linker" as used herein, refers to a nucleic acid or non-nucleic acid linker molecule that is designed as a biodegradable linker to connect one molecule to another molecule, for example, a biologically active molecule to a nucleic acid molecule of the invention. The biodegradable linker is designed such that its stability can be modulated for a particular purpose, such as delivery to a particular tissue or cell type. The stability of a nucleic acid-based biodegradable linker molecule can be modulated by using various chemistries, for example combinations of ribonucleotides, deoxyribonucleotides, and chemically-modified nucleotides, such as 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example, an oligonucleotide of about 2, 3, 4, 5, 6, 7, 8, 9, 10, 11, 12, 13, 14, 15, 16, 17, 18, 19, or 20 nucleotides in length, or can comprise a single nucleotide with a phosphorus-based linkage, for example, a phosphoramidate or phosphodiester linkage. The biodegradable nucleic acid linker molecule can also comprise nucleic acid backbone, nucleic acid sugar, or nucleic acid base modifications.

The term "biodegradable" as used herein, refers to degradation in a biological system, for example enzymatic degradation or chemical degradation.

The term "phospholipids" as used herein, refers to a hydrophobic molecule comprising at least one phosphorus group. For example, a phospholipid can comprise a phosphorus-containing group and saturated or unsaturated alkyl group, optionally substituted with OH, COOH, oxo, amine, or substituted or unsubstituted aryl groups.

Nucleic acid molecules according to the present invention which are delivered exogenously optimally are stable within cells until reverse transcription of the RNA has been modulated long enough to reduce the levels of the RNA transcript. The nucleic acid molecules are resistant to nucleases in order to function as effective intracellular therapeutic agents or diagnostic agents or diagnostic means. Improvements in the chemical synthesis of nucleic acid molecules described in the instant invention and in the art have expanded the ability to modify nucleic acid molecules by introducing nucleotide modifications to enhance their nuclease stability as described above.

In yet another embodiment, siNA molecules having chemical modifications that maintain or enhance enzymatic activity of proteins involved in RNAi are provided. Such nucleic acids are also generally more resistant to nucleases than unmodified nucleic acids. Thus, *in vitro* and/or *in vivo* the activity should no the significantly lowered.

Irrespective of these various designs of siRNA, it will be acknowledged by the ones skilled in the art that according to their origin or function, three types of naturally occurring small RNA have been described: short interfering RNAs (siRNAs), repeat-associated short interfering RNAs (rasiRNAs) and microRNAs (miRNAs). In nature, dsRNA can be produced by RNA-templated RNA polymerization (for example, from viruses) or by hybridization of overlapping transcripts (for example, from repetitive sequences such as transgene arrays or transposons). Such dsRNAs give rise to siRNAs or rasiRNAs, which generally guide mRNA degradation and/or chromatin modification. In addition, endogenous transcripts that contain complementary or near-complementary 20- to 50-base-pair inverted repeats fold back on themselves to form dsRNA hairpins. These dsRNAs are processed into miRNAs that mediate translational repression, although they may also guide mRNA degradation. Finally, artificial introduction of long dsRNAs or siRNAs has been adopted as a tool to inactivate gene expression, both in cultured cells and inliving organisms (Meister & Tuschl, 2004, Nature, 431: 343-349.)

Designing the nucleic acid molecules to be detected in accordance with the methods of the present invention, as L-nucleic acid is advantageous for several reasons. L-nucleic acids are enantiomers of naturally occurring nucleic acids. D-nucleic acids, however, are not very stable in aqueous solutions and particularly in biological systems or biological samples due to the widespread presence of nucleases. Naturally occurring nucleases, particularly nucleases from animal cells are not capable of degrading L-nucleic acids. Because of this the biological half-life of the L-nucleic acid is significantly increased in such a system, including the animal and human body. Due to the lacking degradability of L-nucleic acid no nuclease degradation products are generated and thus no side effects arising therefrom observed. This aspect delimits the L-nucleic acid of factually all other compounds which are used in the therapy of diseases and/or disorders involving the presence of a target molecule against which the nucleic acid molecule to be detected in accordance with the methods of the present invention, are directed. L-nucleic acids which specifically bind to a target molecule through a mechanism different from Watson Crick base pairing, or aptamers which consists partially or completely of L-nucleotides, particularly with those parts of the aptamer being involved in the binding of the aptamer to the target molecule, are also called spiegelmers and, e. g. described in international patent EP1135527.

It is within the present invention that the nucleic acids disclosed herein comprise a moiety which preferably is a high molecular weight moiety and/or which preferably allows to modify the characteristics of the nucleic acid in terms of, among others, residence time in the animal body, preferably the human body. A particularly preferred embodiment of such modification is PEGylation and HESylation of the nucleic acids according to the present invention. As used herein PEG stands for poly(ethylene glycole) and HES for hydroxyethly starch. PEGylation as preferably used herein is the modification of a nucleic acid according to the present invention whereby such modification consists of a PEG moiety which is attached to a nucleic acid according to the present invention. HESylation as preferably used herein is the modification of a nucleic acid according to the present invention whereby such modification consists of a HES moiety which is attached to a nucleic acid according to the present invention. These modifications as well as the process of modifying a nucleic acid using such modifications, is described in European patent application EP 1 306 382, the disclosure of which is herewith incorporated in its entirety by reference. Such embodiment is particularly preferred when the nucleic acid binds to an extra-cellular target, and whereby such nucleic acid is an aptamer or a Spiegelmer.

Preferably, the molecular weight of a modification consisting of or comprising a high molecular weight moiety is about from 2,000 to 200,000 Da, preferably 20,000 to 120,000 Da, particularly in case of PEG being such high molecular weight moiety, and is preferably about from 3,000 to 180,000 Da, more preferably from 5,000 to 130,000 Da, particularly in case of HES being such high molecular weight moiety. The process of HES modification is, e.g., described in German patent application DE 1 2004 006 249.8 the disclosure of which is herewith incorporated in its entirety by reference.

It is within the present invention that either of PEG and HES may be used as either a linear or branched from as further described in the patent applications WO2005074993 and PCT/EP02/11950. Such modification can, in principle, be made to the nucleic acid molecules of the present invention at any position thereof. Preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule of the present invention either directly or through a linker. It is also within the present invention that the nucleic acid molecule according to the present invention comprises one or more modifications, preferably one or more PEG and/or HES moiety. In an embodiment the individual linker molecule attaches more than one PEG moiety or HES moiety to a nucleic acid molecule according to the present invention. The linker used in connection with the present invention can itself be either linear or branched. This kind of linkers are known to the ones skilled in the art and are further described in the patent applications WO2005074993 and PCT/EP02/11950.

Without wishing to be bound by any theory, it seems that by modifying the nucleic acids according to the present invention with high molecular weight moiety such as a polymer and more particularly the polymers disclosed herein, which are preferably physiologically acceptable, the excretion kinetic is changed. More particularly, it seems that due to the increased molecular weight of such modified inventive nucleic acids and due to the nucleic acids not being subject to metabolism particularly when in the L form, excretion from an animal body, preferably from a mammalian body and more preferably from a human body is decreased. As excretion typically occurs via the kidneys, the present inventors assume that the glomerular filtration rate of the thus modified nucleic acid is significantly reduced compared to the nucleic acids not having this kind of high molecular weight modification which results in an increase in the residence time in the body. In connection therewith it is particularly noteworthy that, despite such high molecular weight modification the specificity of the nucleic acid according to the present invention is not affected in a detrimental manner. Insofar, the nucleic acids according to the present invention have surprising characteristics - which normally cannot be expected from pharmaceutically active compounds - such that a pharmaceutical formulation providing for a sustained release is not necessarily required to provide for a sustained release. Rather the nucleic acids according to the present invention in their modified form comprising a high molecular weight moiety, can as such already be used as a sustained release-formulation. Insofar, the modification(s) of the nucleic acid molecules as disclosed herein and the thus modified nucleic acid molecules and any composition comprising the same may provide for a distinct, preferably controlled pharmacokinetics and biodistribution thereof. This also includes residence time in circulation and distribution to tissues. Such modifications are further described in the patent application PCT/EP02/11950.

However, it is also within the present invention that the nucleic acids disclosed herein do not comprise any modification and particularly no high molecular weight modification such as PEGylation or HESylation. Such embodiment is particularly preferred when the nucleic acid binds to mRNA like RNAi mediating molecules, antisense molecules or ribozymes or when the target is an intracellular target such as for decoy oligonucleotides.

Upon a hybridisation a double-stranded structure is formed. It will be acknowledged by the one skilled in the art that such hybridisation may or may not occur, particularly under in vitro and/or in vivo conditions. Also, in case of such hybridisation, it is not necessarily the case that the hybridisation occurs over the entire length of the two stretches where, at least based on the rules for base pairing, such hybridisation and thus formation of a double-stranded structure may occur. As preferably used herein, a double-stranded structure is a part of a molecule or a structure formed by two or more separate strands, whereby at least one, preferably two or more base pairs exist which are base pairing preferably in accordance with the Watson-Crick base pairing rules. It will also be acknowledged by the one skilled in the art that other base pairing such as Hoogsten base pairing may exist in or form such double-stranded structure.

It is also within the present invention that the inventive nucleic acids, regardless whether they are present as D-nucleic acids, L-nucleic acids or D,L-nucleic acids or whether they are DNA or RNA, may be present as single stranded or double stranded nucleic acids. Typically, the inventive nucleic acids are single stranded nucleic acids which exhibit defined secondary structures due to the primary sequence and may thus also form tertiary structures. The inventive nucleic acids, however, may also be double stranded in the meaning that two strands which are complementary or partially complementary to each other are hybridised to each other. This confers stability to the nucleic acid which, in particular, will be advantageous if the nucleic acid is present in the naturally occurring D-form rather than the L-form.

Such a label is preferably selected from the group comprising radioactive, enzymatic and fluorescent labels. In principle, all known assays developed for antibodies can be adopted for the nucleic acids subject to the methods according to the present invention whereas the target-binding antibody is substituted to a target-binding nucleic acid. In antibody-assays using unlabeled target-binding antibodies the detection is preferably done by an secondary antibody which is modified with radioactive, enzymatic and fluorescent labels and bind to the target-binding antibody at its Fc-fragment. In the case of a nucleic acid, preferably a nucleic acid according to the present invention, the nucleic acid is modified with such a label, whereby preferably such a label is selected from the group comprising biotin, Cy-3 and Cy-5, and such label is detected by an antibody directed against such label, e.g. an anti-biotin antibody, an anti-Cy3 antibody or an anti-Cy5 antibody, or - in the case that the label is biotin - the label is detected by streptavidin or avidin which naturally bind to biotin. Such antibody, streptavidin or avidin in turn is preferably modified with a respective label, e.g. a radioactive, enzymatic or fluorescent label (like an secondary antibody).

In a further embodiment the nucleic acid molecules subject to the methods according to the present invention are detected or analysed by a second detection means, wherein the said detection means is a molecular beacon. The methodology of molecular beacon is known to persons skilled in the art. In brief, nucleic acids probes which are also referred to as molecular beacons, are a reverse complement to the nucleic acids sample to be detected and hybridise because of this to a part of the nucleic acid sample to be detected. Upon binding to the nucleic acid sample the fluorophoric groups of the molecular beacon are separated which results in a change of the fluorescence signal, preferably a change in intensity. This change correlates with the amount of nucleic acids sample present.

The detection step inherent to the methods of the present invention may also involve the use of a second detection means which is, preferably, also selected from the group comprising nucleic acids, polypeptides, proteins and embodiments in the various embodiments described herein. Such detection means are preferably specific for the nucleic acid subject to the methods according to the present invention. In a more preferred embodiment, the second detection means is a molecular beacon. Either the nucleic acid or the second detection means or both may comprise in a preferred embodiment a detection label. The detection label is preferably selected from the group comprising biotin, a bromo-desoxyuridine label, a digoxigenin label, a fluorescence label, a UV-label, a radio-label, and a chelator molecule. Alternatively, the second detection means interacts with the detection label which is preferably contained by, comprised by or attached to the nucleic acid. Particularly preferred combinations are as follows:
Finally, it is also within the present invention that the second detection means is detected using a third detection means, preferably the third detection means is an enzyme, more preferably showing an enzymatic reaction upon detection of the second detection means, or the third detection means is a means for detecting radiation, more preferably radiation emitted by a radionuclide. Preferably, the third detection means is specifically detecting and/or interacting with the second detection means.

Preferably the derivative of the nucleic acid comprises at least one fluorescent derivative of adenosine replacing adenosine. In a preferred embodiment the fluorescent derivative of adenosine is ethenoadenosine.

In a preferred embodiment of each of the various aspects of the present invention, the methods of the present invention are performed in 96-well plates, where components and in particular the capture probe are immobilized in the reaction vessels as described above and the wells acting as reaction vessels.

It will be acknowledged that typically the capture probe, in its diverse embodiments, as well as and in particular the detection probe are provided with a detection means. Such detection means are preferably tags and labels. Preferably, a tag is used to mediate the attachment, typically covalent or non-covalent to another moiety, compound or a support. Preferably a label is used for detection purpose of a compound, moiety or entity which carries such label.

As will be acknowledged by the one skilled in the art, the methods according to the first aspect of the present invention can be carried by the use of a capture probe consisting of D-ribonucleotides that are a substrate for single-stranded RNA specific nucleases, e.g. RNAse I and RNAseII, RNAse T1, RNAse A) or by the use of a capture probe consisting of D-desoxyribonucleotides that are a substrate for single-stranded DNA specific nucleases, e.g. MungBeanNuclease.

It is also within the present invention that it is related in a further aspect to a kit, whereby such kit can preferably be used in the performing of any of the methods according to the present invention. Accordingly, such kit may comprise the various probes described herein. More specifically, it typically comprises at least one capture probe and one detection probe and optionally one or several bridging probes and, any encymatic activities required for the performing of the methods according to the present invention and also preferably an instruction leaflet on how to use the various ingredients so as to practice the method subject to the present application.

The various SEQ.ID. Nos., the chemical nature of the nucleic acid molecules according to the present invention as used herein, the actual sequence thereof and the internal reference number is summarized in the following table.

| **Seq.-ID** | **RNA/DNA** | **Sequence** | **Internal Reference** |
|---|---|---|---|
| 1 | D-DNA | 5'-P-GATGGCGCCACTCCGTCGTG-(Spacer18)₂-3'-NH₂ | siRNA capture probe |
| 2 | D-RNA | 5'-Biotin-C18_Spacer-GCGCCAUC-Sequence_of_the_first_siRNA_strand | siRNA bridge |
| 3 | L-RNA | 5'-GGCGACAUUGGUUGGGCAUGAGGCGAGGCCCUUUGAUGAAUCCGCGGCCA | mNOX-E36 |
| 4 | L-RNA | 5'-GGCGACAUUGGUUGGGCAUGAGGCGAGGCCCUUUGAUGAAUCCGCGGCCA-3'40 kDa-PEG | mNOX-E36-3'-PEG |
| 5 | L-DNA | 5'-CCAATGTCGCC-(Spacer18)₂-NH4⁺ -3' | mNOX-E36 capture probe |
| 6 | L-RNA | 5'- Biotin-(Spacer18)₂-CGCAGAGCC | mNOX-E36 detection probe |
| 7 | L-RNA | 5'-GCACGUCCCUCACCGGUGCAAGUGAAGCCGUGGCUCUGCG | NOX-36 |
| 8 | L-RNA | 5'40 kDa-PEG -GCACGUCCCUCACCGGUGCAAGUGAAGCCGUGGCUCUGCG | NOX-36-5'-PEG |
| 9 | L-RNA | 5'-GCACGUCCCUCACCGGUGCAAGUGAAGCCGUGGCUCUGCG-3'40 kDa-PEG | NOX-36-3'-PEG |
| 10 | L-DNA | 5'-GAGGGACGTGC-(Spacer18)₂-NH₂ -3' | NOX-E36 capture probe |
| 11 | L-RNA | 5'- Biotin-(Spacer18)₂-CGCAGAGCC | NOX-E36 detection probe |
| 12 | L-RNA | 5'-GCGUGGUGUGAUCUAGAUGUAUUGGCUGAUCCUAGUCAGGUACGC | NOX-A12 |
| 13 | L-RNA | 5'-40 kDa-PEG-GCGUGGUGUGAUCUAGAUGUAUUGGCUGAUCCUAGUCAGGUACGC | NOX-A12-5'-PEG |
| 14 | L-RNA | 5'-GCGUGGUGUGAUCUAGAUGUAUUGGCUGAUCCUAGUCAGGUACGC-3'-40 kDa-PEG | NOX-A12-3'-PEG |
| 15 | L-DNA | 5'-GATCACACCACGC-(C18-PEG-spacer)-(C18-PEG-spacer)-NH₂-3' | NOX-A12 capture probe |
| 16 | L-RNA | 5'-Biotin-(C18-PEG-spacer)-(C18-PEG-spacer)-GCGUACCUGAC | NOX-A12 detection probe |

It will also be understood by the ones skilled in the art that the terms detect probe and detection probe are used in an interchangeable manner. Also, the terms bridge, c-bridge, d-bridge and bridging probe are used in an interchangeable manner.

The present invention is further illustrated by the figures, examples and the sequence listing from which further features, embodiments and advantages may be taken, wherein more specifically,
- Figs. 1 and 2: show schematically the method according to the first aspect, with a detailed description in Example 1;
- Fig. 3: shows schematically the method according to the second aspect, with a detailed description in Example 2;
- Fig. 4: shows schematically the method according to the third aspect , with a detailed description in Example 3;
- Fig. 5: shows schematically the method according to the fourth aspect , with a detailed description in Example 4;
- Fig. 6: shows schematically the method according to the fifth aspect, with a detailed description in Example 5;
- Fig. 7: shows schematically the method according to the sixth aspect., with a detailed description in Example 6;
- Fig. 8: shows the detection of the RNAi which consists of D-RNA building blocks, 2'-O-Methyl-modified RNA building blocks and phosphorothioate building blocks,. and
- Fig. 9: shows the pharmacokinetics of Spiegelmers mNOX-E36 and mNOX-E36-3'-PEG in plasma during the study, indicated as plasma concentration of Spiegelmer mNOX-E36 / mNOX-E36-3'-PEG as a function of time;

### Example 1: RNAi detection assay based on an RNAse protection assay

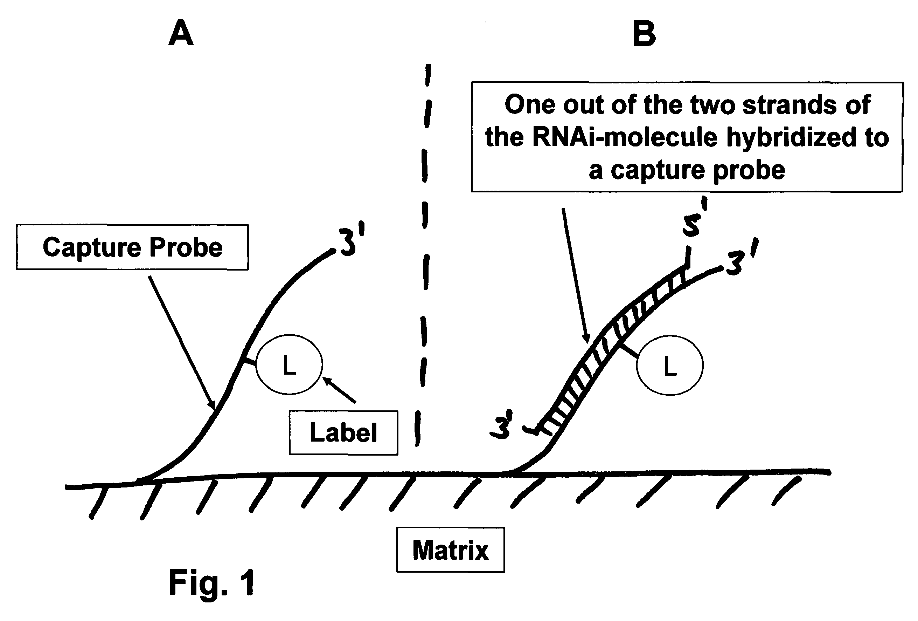

Within the described RNAi detection assay a capture probe is used whereby its sequence shares the complete complementary sequence or parts of the complementary sequence of one out of the two strands of the RNAi molecule. Moreover, the complementary sequence of the one out of the two strands of the RNAi molecule to be measured can be flanked by additional nucleotides. Using a capture probe comprising the complete complementary sequence of the one out of the two strands of the RNAi molecule allows high specificity and selectivity between the RNAi molecules to be measured and other RNAi molecules and/ or RNA structures occurring in samples that are analyzed. The sequence stretch of the capture probe that is complementary to the RNAi molecule is formed by D-RNA nucleotides that are not resistant to RNAses or by D-DNA nucleotides that are not resistant to single-stranded specific DNAses.. The capture probe is immobilised to a matrix or surface (e.g. a 96-well plate) by covalent or non-covalent binding whereby the capture probe can be immobilised directly at one of its ends or via a linker between of one of its ends and the surface or matrix. The linker can be formed by hydrophilic linkers of skilled in the art or by D-DNA nucleotides, modified D-DNA nucleotides, in regard to RNAse resistance modified D-RNA nucleotides, D-LNA nucleotides, PNA nucleotides, L-RNA nucleotides, modified L-RNA nucleotides, L-DNA nucleotides, modified L-DNA nucleotides and/or L-LNA nucleotides. However, all kind of linkers or nucleotides used as linker have to be fully resistant to RNAses. Moreover the capture probe carries a marker molecule or label that can be detected. The label can be linked to each nucleotide of the sequence stretch that is complementary to the RNAi molecule. Preferably, the label is linked to one of the central nucleotides of the sequence stretch within the capture probe that is complementary to the RNAi molecule (Fig. 1A).

The detection of RNAi molecules to be measured can be carried out as follows:
The double-strands of the RNAi molecule are separated from each other (e.g. by a denaturation step) in presence or absence of the capture probe and if needed are given to the immobilised capture probe. The one out of the two strands of the RNAi molecule to be measured hybridises completely or partly to the capture probe consisting of D-RNA nucleotides as depicted in Fig. 1B and a stretch of double-stranded RNA is formed. If a single-stranded specific RNAse (e.g. RNase T1, RNase A) is added, the capture probe molecules that are not hybridised to the one out of the two strands of the RNAi molecule to be measured are degraded by nuclease digestion leading to a release of the label or marker molecule. The label or marker and the RNA fragments and/or nucleotides can be removed by several washing steps. The amount of intact (carrying a label or marker molecule) and immobilised capture probe can be measured and compared to a control which comprise the same amount of capture probe that was present before RNA digestion. Calculating from the reduction of the signal in comparison to the control, the amount of the bound RNAi strand (and therefore RNAi at all) can be determined.

The described RNAi detection assay can be done either for the first or second strand out of the two strands of the RNAi molecule. Alternatively, both strands out of the two strands of the RNAi molecule can be detected in parallel reaction batches using the respective designed capture probes. In order to quantify the amount of the RNAi at all, the amount of both strands should be measured. The analysis should be lead to more or less equal amounts of the first and the second strand of the RNAi molecule.

### Example 2: RNAi detection assay based on an sandwich hybridisation assay

Within the described RNAi detection assay a capture probe and a detect probe are used. The capture probe shares the first part and the detect probe the second part of the complementary sequence of one out of the two strands of an RNAi molecule to be measured. Both, capture and detect probe, can be formed by D-DNA nucleotides, modified D-DNA nucleotides, modified D-RNA nucleotides, D-RNA nucleotides, D-LNA nucleotides and/or PNA nucleotides.

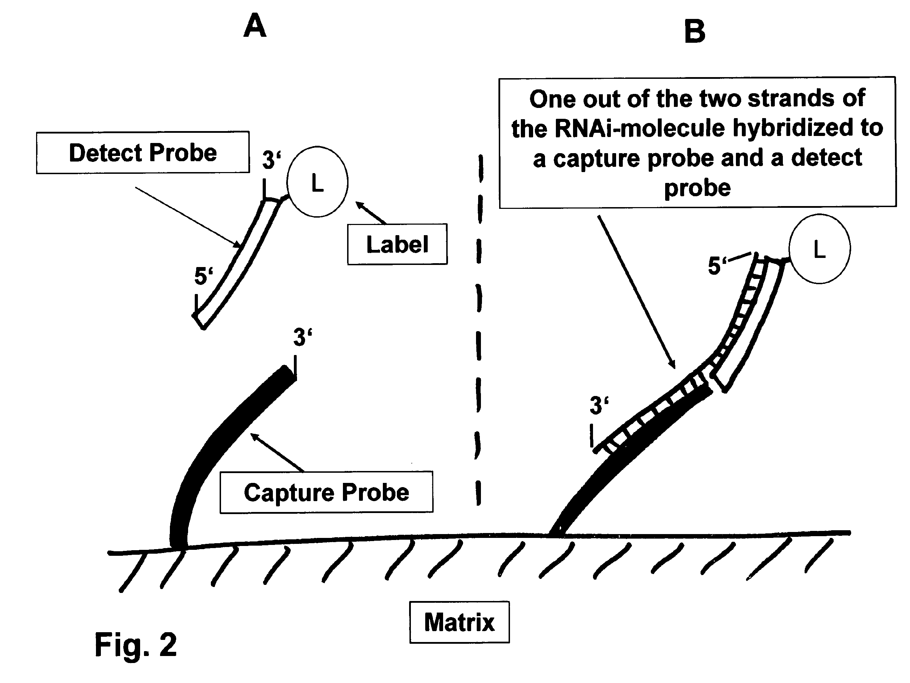

Hence, the capture probe comprise a sequence stretch complementary to the 5'-end of the one out of the two strands of the RNAi molecule and the detect probe comprise a sequence stretch complementary to the 3'-end of the one out of the two strands of the RNAi molecule. In this case the capture probe is immobilised to a surface or matrix via its 5'-end whereby the capture probe can be immobilised directly at its 5'-end or via a linker between of its 5'-end and the surface or matrix. However, in principle the linker can be linked to each nucleotide of the capture probe. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides (Fig. 2A).

Alternatively, the capture probe comprises a sequence stretch complementary to the 3'-end of the one out of the two strands of the RNAi molecule and the detect probe comprise a sequence stretch complementary to the 5'-end of the one out of the two strands of the RNAi molecule. In this case the capture probe is immobilised to a surface or matrix via its 3'-end whereby the capture probe can be immobilised directly at its 3'-end or via a linker between of its 3'-end and the surface or matrix. However, in principle the linker can be linked to each nucleotide of the sequence stretch that is complementary to the RNAi molecule to be measured. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

The number of nucleotides of the capture and detect probe that may hybridise to one out of the two strands of the RNAi molecule to be measured is variable and can be dependant from the number of nucleotides of the capture and/or the detect probe and/or the RNAi molecule itself. The total number of nucleotides of the capture and the detect probe that may hybridise to one out of the two strands of the RNAi molecule should be maximal the number of nucleotides that are comprised by the one out of the two strands of the RNAi molecule to be measured. The minimal number of nucleotides (2 to 10 nucleotides) of the detect and capture probe should allow hybridisation to the 5'-end or 3'-end, respectively, of the one out of the two strands of the RNAi molecule. In order to realize high specificity and selectivity between the RNAi molecules and other RNAi molecules and/ or RNA structures occurring in samples that are analyzed the total number of nucleotides of the capture and detect probe should be or maximal the number of nucleotides that are comprised by the one out of the two strands of the RNAi molecule.

Moreover the detect probe carries a marker molecule or label that can be detected. The label or marker molecule can in principle be linked to each nucleotide. Preferably, the label or marker is located at the 5'-end or 3'-end of the detect probe whereby between the nucleotides within the detect probe that are complementary to the RNAi molecule and the label a linker can be inserted. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides (Fig. 2A).

The detection of RNAi molecules can be carried out as follows:
The double-strands of the RNAi molecule are separated from each other (e.g. by a denaturation step) in presence or absence of the capture probe and/or the detect probe and if needed are given to the immobilised capture probe and/ or detect probe. The one out of the two strands of the RNAi molecule to be measured hybridises with one of its ends to the capture probe and with the other end to the detect probe as depicted in Fig. 2B. Afterwards unbound detect probe are removed by several washing steps. The amount of bound capture (carrying a label or marker molecule) can be measured. Calculating from the amount of signal the amount of the bound RNAi strand (and therefore RNAi at all) can be determined.

The described RNAi detection assay can be done either for the first or second strand out of the two strands of the RNAi molecule. Alternatively, both strands out of the two strands of the RNAi molecule can be detected in parallel reaction batches using the respective designed capture probes. In order to quantify the amount of the RNAi at all, the amount of both strands should be measured. The analysis should be lead to more or less equal amounts of the first and the second strand of the RNAi molecule.

### Example 3: RNAi detection assay based on a competition assay

Within the described RNAi detection assay a capture probe and a detect probe are used. Both, capture and detect probe, can be formed by D-DNA nucleotides, modified D-DNA nucleotides, modified D-RNA nucleotides, D-RNA nucleotides, D-LNA nucleotides and/or PNA nucleotides. The detect probe shares a sequence stretch that is homolog to one out of the two strands of the RNAi molecule to be measured.

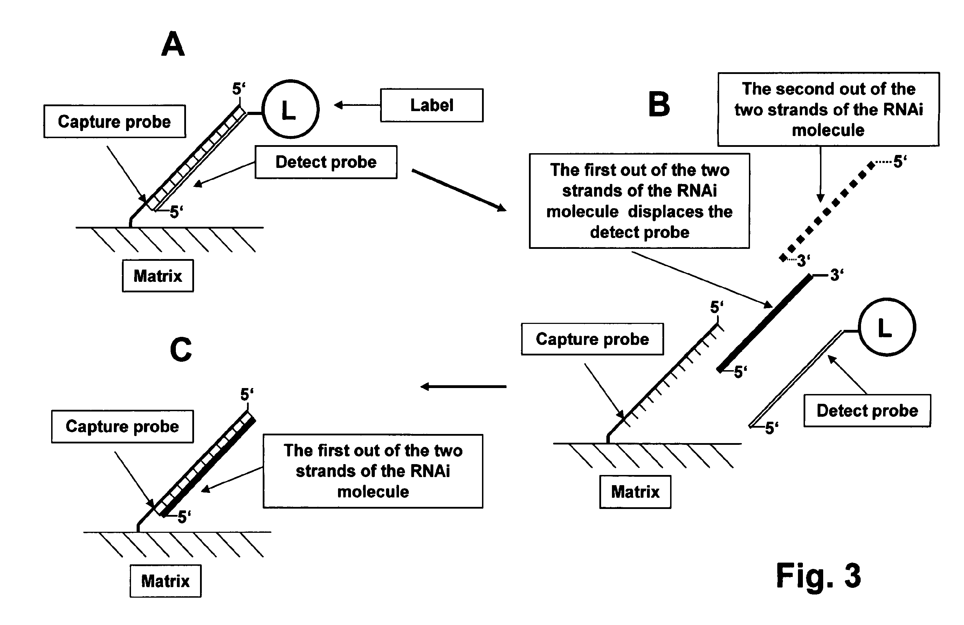

The capture probe comprises a sequence stretch that is complementary the one out of the two strands of the RNAi molecule to be measured. The capture probe can be immobilised to a surface or matrix directly at one of its ends to a surface or matrix or via a linker between of one of its ends the surface or matrix (Fig. 3A). However, in principle the linker can be linked to each nucleotide of the capture probe. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

The detect probe carries a marker molecule or label that can be detected (Fig. 3A). The marker molecule or label can in principle be linked to each nucleotide. Preferably, the marker molecule or label is located at the 5'-end or 3'-end of the detect probe whereby between the nucleotides within the detect probe that are homolog to one out of the two strands of the RNAi molecule and the label or marker molecule a linker can be inserted. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

The detection of RNAi molecules can be carried out as follows:
The detect probe is as added to the immobilised capture probe and/or the RNAi molecule to be measured. If the detect probe is added to the capture probe the sequence stretch of the capture probe that is complementary to the detect probe will hybridise to the fully detect probe (Fig. 3A). Then RNAi molecules are added and the sample is denatured (e.g. by heat). As consequence the two strands of the double-stranded RNAi molecule are separate from each other and the detect probe is separated from the capture probe. Preferably, the detect probe is given in advance to the RNAi molecules and this mixture is denatured. As consequence the two strands of the double-stranded RNAi molecule are separate from each other and the detect probe binds to one out of the two strands of the RNAi molecule to measured. Initiation of renaturation process (e.g. cooling, if the denaturation step was done by heat) can lead to re-hybridisation of detect and capture probe, of the detect probe and the one out of the strands of the RNAi molecule that is complementary to the detect probe, and of the capture probe and one out of the strands of the RNAi molecule that is complementary to the capture probe (and is homolog to the detect probe). The crucial point is thereby that the one out of the strands of the RNAi molecule that is complementary to the capture probe competes with the detect probe for binding to the capture probe (Fig. 3B). The detect probe that binds to the one out of the strands of the RNAi molecule that is complementary to the detect probe is removed by several washing step. Dependant on the amount of RNAi molecules to be analysed, molecules of the detect probe are replaced by molecules of the one out of the strands of the RNAi molecule that is complementary to the capture probe (Fig. 3C).

The amount of remained detect probe (bound to the capture probe) can be measured and compared to a control which comprise the same amount of detect probe that was present before adding the RNAi molecules. Calculating from the reduction of the signal in comparison to the control, the amount of the bound RNAi strand (and therefore RNAi at all) can be determined.

The described RNAi detection assay can be done either for the first or second strand out of the two strands of the RNAi molecule. Alternatively, both strands out of the two strands of the RNAi molecule can be detected in parallel reaction batches using the respective designed capture probes. In order to quantify the amount of the RNAi at all, the amount of both strands should be measured. The analysis should be lead to more or less equal amounts of the first and the second strand of the RNAi molecule.

### Example 4: An RNAi detection assay based on ligation

Within the described RNAi detection assay comprise a capture probe, a detect probe and two 'bridge' oligonucleotides that are needed for template-directed ligation are used. The capture probe, the detect probe and the two 'bridge' oligonucleotides can be formed by D-DNA nucleotides, modified D-DNA nucleotides, modified D-RNA nucleotides and D-RNA nucleotides.

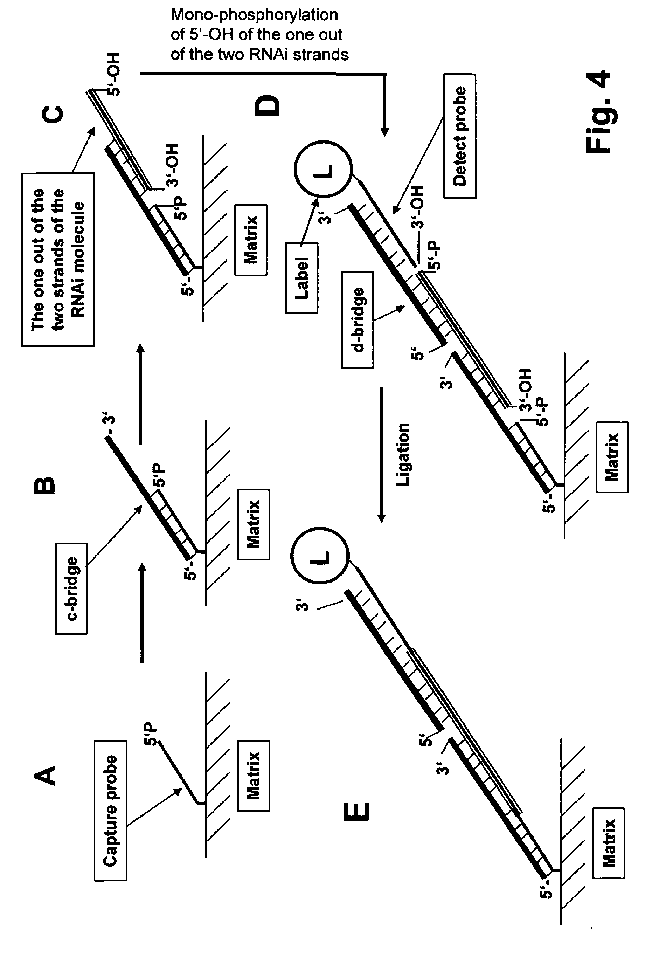

The capture probe shares a stretch at its 5'-end with a minimal number of nucleotides that allows hybridisation to a bridge oligonucleotide that is used for template-directed ligation, herein referred as 'c-bridge'. Therefore the stretch of nucleotides at the 5'-end of the capture comprises two to 20 nucleotides, preferably six to 15 nucleotides. Additionally, at its 5'-end the capture probe carries a 5'-monophosphate that allows the ligation to an oligonucleotide that carries a 3'-OH (Fig. 4A). The c-bridge comprises at its 5'-end a stretch of nucleotides with a minimal number of nucleotides that allows hybridisation to the 5'-end of the capture probe. Therefore the stretch of nucleotides at the 5'-end of the c-bridge comprise two to 20 nucleotides, preferably six to 15 nucleotides. Moreover, the c-bridge comprises at its 3'-end 3' of the stretch that is used for hybridisation to the capture probe an additional stretch that can hybridize to the 3'-end of one out of the two strands of the RNAi molecule to be measured. Therefore the stretch of nucleotides at the 3'-end of c-bridge comprise two to 20 nucleotides, preferably 7 to 11 nucleotides (Fig. 4B).

The capture probe can be immobilised directly at one of its 3'-end to a surface or matrix or via a linker between of one of its 3'-end and the surface or matrix. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

The detect probe comprises a minimal number of nucleotides that allows hybridisation to a bridge oligonucleotide that are used for template-directed ligation, herein referred as 'd-bridge'. Therefore the detect probe comprise two to 20 nucleotides, preferably 7 to 15 nucleotides. The d-bridge comprise at its 3'-end a stretch of nucleotides with a minimal number of nucleotides that allows hybridisation to the detect probe. Therefore the stretch of nucleotides at the 3-end of the c-bridge comprise two to 20 nucleotides, preferably 7 to 15 nucleotides. Moreover d-bridge comprise at its 5'-end 5' of the stretch that is used for hybridisation to the detect probe and an additional stretch that can hybridize to the 5'-end of one out of the two strands of RNAi molecules to be measured. Therefore the stretch of nucleotides at the 5'-end of d-bridge comprise two to 20 nucleotides, preferably seven to 11 nucleotides (Fig. 4D).

The detect probe comprises a marker molecule or label that can be detected. The label can in principle be linked to each nucleotide. Preferably, the label is located at the 5'-end of the detect probe whereby between 5'-end of the detect probe and the label a linker can be inserted. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides (Fig. 4D).

Alternatively, instead of the c-bridge and the d-bridge one c-d-bridge can be used. The c-d-bridge is formed by nucleotides as described for the c- or d-bridge. As described for the c-bridge the c-d-bridge can be immobilised to a matrix or surface. The c-d bridge comprise the sequence stretches as described for the c- and d-bridge including the fully complementary sequence of the one out of the strands of the RNAi molecule to be measured.

The detection of RNAi molecules can be carried out as follows:
At first the capture probe is immobilised to a surface or matrix (Fig. 4A). Secondly, the c-bridge is added and hybridise to the capture probe forming a double-stranded oligonucleotide with whereby the nucleotides of the 3'-end of the c-bridge are not paired to other nucleotides and are free to hybridize to the 3'-end of the one out of the two strands of the RNAi molecule to be measured (Fig. 4B). In the following the RNAi molecule that a short time before was denatured leading to two separated strands of the RNAi molecule to be measured is added (Fig. 4C). Alternatively, the denaturation can be done in presence of RNAi molecule and c-bridge or RNAi molecule, c-bridge and capture probe. A further alternative is that the double-strands of the RNAi molecule were separated from each other in advance (e.g. by a denaturation step) and one out of the two strands of the RNAi molecule were taken out (e.g. by hybridisation to a complementary oligonucleotide) and the second strand is given to the immobilised capture probe.

The one out of the two strands of the RNAi molecule to be measured hybridise to the 3'-end of c-bridge (Fig. 4C). After one or several washing steps the 5'-OH of the strand of the RNAi molecule is mono-phosphorylated (e.g. using T4 Kinase). After one or several washing steps in order to remove reactants, the detect probe and d-bridge are added whereby the detect probe and d-bridge can be present as a double-stranded oligonucleotide whereby the complete detect probe hybridize to the 3'-end of d-bridge. The free nucleotides of 5'-end of d-bridge can now hybridize to the 5'-end of the strand of RNAi molecule (Fig. 4D). Due to the hybridisation of the 5'-end of the strand of the RNAi molecule to the 5'-end of c-bridge and due to the hybridisation of the 3'-end of the strand of the RNAi molecule to the 3'-end of d-bridge, the 3'-OH of d-bridge and the 5'-monophosphate of the strand of the RNAi molecule or rather the 5'-monophosphate of c-bridge and the 3'-OH of the strand of the RNAi molecule are brought together and can be linked by ligation using DNA ligase (Fig. 4E). If the one out of the two strands of the RNAi molecule to be measured miss at its 5'- and/or 3'-end one or more nucleotides, no ligation reaction at its 5'- and/or 3'-end can occur. These molecules of the one out of the two strands of the RNAi are not detected, because they are not linked to detect probe or/and to the capture and therefore no labelled or/and exist no longer in the reaction batch after the washing. After several washing steps the amount of bound detect probe (carrying a label or marker molecule) can be measured. Calculating from the amount of signal the amount of the bound RNAi strand (and therefore RNAi at all) can be determined. The described RNAi detection assay can be done either for the first or second strand out of the two strands of the RNAi molecule. Alternatively, both strands out of the two strands of the RNAi molecule can be detected in parallel reaction batches using the respective designed capture probes, detect probes, c-bridges and d-bridges. In order to quantify the amount of the RNAi at all, the amount of both strands should be measured. The analysis should be lead to more or less equal amounts of the first and the second strand of the RNAi molecule.

The assay as described above can be also carried as described in the following. In this case used capture probe, detect probe, c-bridge and d-bridge are arranged in an alterative fashion.

The capture probe shares a stretch at its 3'-end with a minimal number of nucleotides that allows hybridisation to a bridge oligonucleotide that is used for template-directed ligation, herein referred as `c-bridge'. Therefore the stretch of nucleotides at the 3'-end of the capture comprises two to 20 nucleotides, preferably 7 to 15 nucleotides. The c-bridge comprise at its 3'-end a stretch of nucleotides with a minimal number of nucleotides that allows hybridisation to the 3'-end of the capture probe. Therefore the stretch of nucleotides at the 3'-end of the c-bridge comprise two to 20 nucleotides, preferably 7 to 15 nucleotides. Moreover, the c-bridge comprises at its 5'-end 5' of the stretch that is used for hybridisation to the capture probe and an additional stretch that can hybridize to the 5'-end of one out of the two strands of the RNAi molecule to be measured. Therefore the stretch of nucleotides at the 5'-end of c-bridge comprise two to 20 nucleotides, preferably 7 to 11 nucleotides.

The capture probe can be immobilised directly at one of its 5'-end to a surface or matrix or via a linker between of one of its 5-end and the surface or matrix. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

The detect probe comprises a minimal number of nucleotides that allows hybridisation to a bridge oligonucleotide that are used for template-directed ligation, herein referred as 'd-bridge'. Therefore the detect probe comprise two to 20 nucleotides, preferably 7 to 15 nucleotides. The d-bridge comprise at its 5'-end a stretch of nucleotides with a minimal number of nucleotides that allows hybridisation to the detect probe. Therefore the stretch of nucleotides at the 5'-end of the c-bridge comprise two to 20 nucleotides, preferably seven to 15 nucleotides. Moreover d-bridge comprise at its 3'-end 3' of the stretch that is used for hybridisation to the detect probe and an additional stretch that can hybridize to the 5'-end of one out of the two strands of RNAi molecules that should be measured. Therefore the stretch of nucleotides at the 5'-end of d-bridge comprise two to 20 nucleotides, preferably 7 to 11 nucleotides. Additionally, at its 5'-end the detect probe carries a 5'-monophosphate that allows the ligation to an oligonucleotide that carries a 3'-OH.

The detect probe carries a marker molecule or label that can be detected. The label can in principle be linked to each nucleotide. Preferably, the label is located at the 3'-end of the detect probe whereby between 3'-end of the detect probe and the label a linker can be inserted. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

Alternatively, instead of the c-bridge and the d-bridge one c-d-bridge can be used. The c-d-bridge is formed by nucleotides as described for the c- or d-bridge. As described for the c-bridge the c-d-bridge can be immobilised to a matrix or surface. The c-d bridge comprise the sequence stretches as described for the c- and d-bridge including the fully complementary sequence of the one out of the strands of the RNAi molecule to be measured.

The detection of RNAi molecules can be carried out as described above.

The described RNAi detection assay can be done either for the first or second strand out of the two strands of the RNAi molecule. Alternatively, both strands out of the two strands of the RNAi molecule can be detected in parallel reaction batches using the respective designed capture probes, detect probes, c-bridges and d-bridges. In order to quantify the amount of the RNAi at all, the amount of both strands should be measured. The analysis should be lead to more or less equal amounts of the first and the second strand of the RNAi molecule.

Due to the design of the molecules herein this template-directed ligation strategy allows sequence specific ligation and detection of individual RNAi molecules.

### Example 5: Another RNAi detection assay based on ligation

Within the described RNAi detection assay comprise a capture probe, a detect probe and one 'bridge' oligonucleotide that are needed for template-directed ligation are used. The capture probe, the detect probe and the one 'bridge' oligonucleotide can be formed by D-DNA nucleotides, modified D-DNA nucleotides, modified D-RNA nucleotides and D-RNA nucleotides.

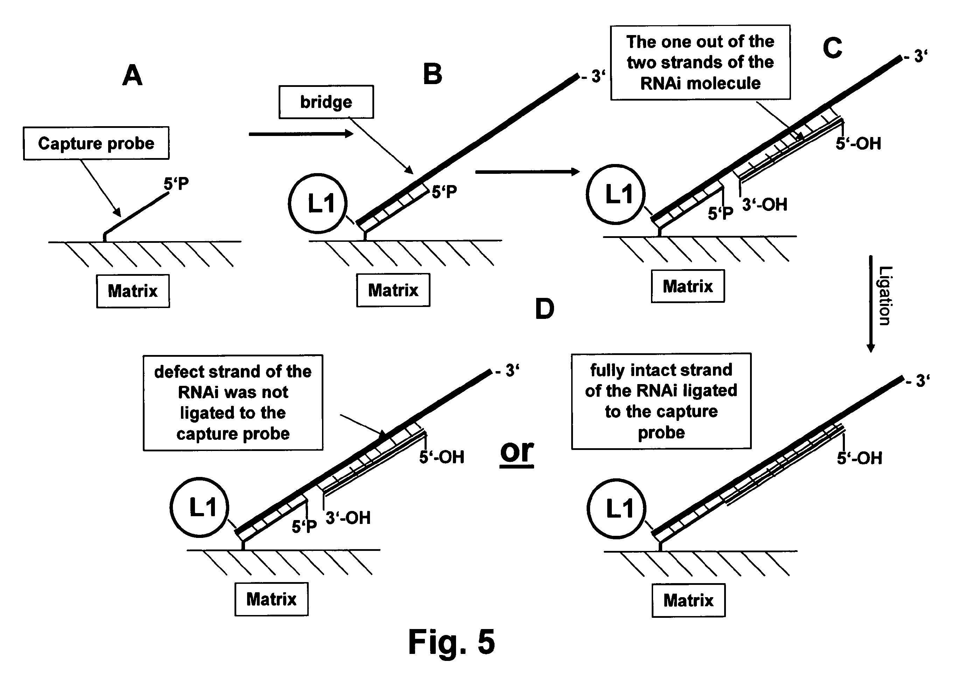

The capture probe comprises stretch at its 5'-end with a minimal number of nucleotides that allows hybridisation to a bridge oligonucleotide that is used for template-directed ligation, herein referred as 'bridge'. Therefore the stretch of nucleotides at the 5'-end of the capture comprises two to 15 nucleotides, preferably six to 10 nucleotides. Additionally, at its 5'-end the capture probe carries a 5'-monophosphate that allows the ligation to an oligonucleotide that carries a 3'-OH (Fig. 5A). The capture probe can be immobilised directly at one of its 3'- end to a surface or matrix or via a linker between of one of its 3'-end and the surface or matrix. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides.

The bridge comprises at its 5'-end a stretch of nucleotides with a minimal number of nucleotides that allows hybridisation to the 5'-end of the capture probe. Therefore the stretch of nucleotides at the 5'-end of the bridge comprise two to 15 nucleotides, preferably six to 10 nucleotides. Moreover, the bridge comprises 3' of the stretch that is used for hybridisation to the capture probe an additional stretch that is complementary one out of the two strands of the RNAi molecule to be measured. Additionally, the sequence stretch at its 3'-end allows the hybridisation of a detect probe. Therefore the stretch of nucleotides at the 3'-end of the bridge comprises two to 20 nucleotides, preferably 10 to 15 nucleotides (Fig. 5B). The bridge carries a marker molecule or label that can be detected. The label can in principle be linked to each nucleotide. Preferably, the label is located at the 5'-end of the bridge whereby between 5'-end of the detect probe and the label a linker can be inserted. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides (Fig. 5B).

Alternatively, instead of one bridge binding to the capture probe, the one out of the two strands of the RNAi molecule and the detect probe two bridges can be used, the c-bridge and the d-bridge. The c-bridge comprises at its 5'-end a stretch of nucleotides with a minimal number of nucleotides that allows hybridisation to the 5'-end of the capture probe. Therefore the stretch of nucleotides at the 5'-end of the c-bridge comprise two to 20 nucleotides, preferably six to 15 nucleotides. Moreover, the c-bridge comprises at its 3'-end 3' of the stretch that is used for hybridisation to the capture probe an additional stretch that can hybridize to the 3'-end of one out of the two strands of the RNAi molecule to be measured. Therefore the stretch of nucleotides at the 3'-end of c-bridge comprise two to 20 nucleotides, preferably 7 to 11 nucleotides. The d-bridge comprises at its 3'-end a stretch of nucleotides with a minimal number of nucleotides that allows hybridisation to the detect probe. Therefore the stretch of nucleotides at the 3-end of the c-bridge comprise two to 20 nucleotides, preferably 7 to 15 nucleotides. Moreover d-bridge comprise at its 5'-end 5' of the stretch that is used for hybridisation to the detect probe and an additional stretch that can hybridize to the 5'-end of one out of the two strands of RNAi molecules to be measured. Therefore the stretch of nucleotides at the 5'-end of d-bridge comprise two to 20 nucleotides, preferably seven to 11 nucleotides.

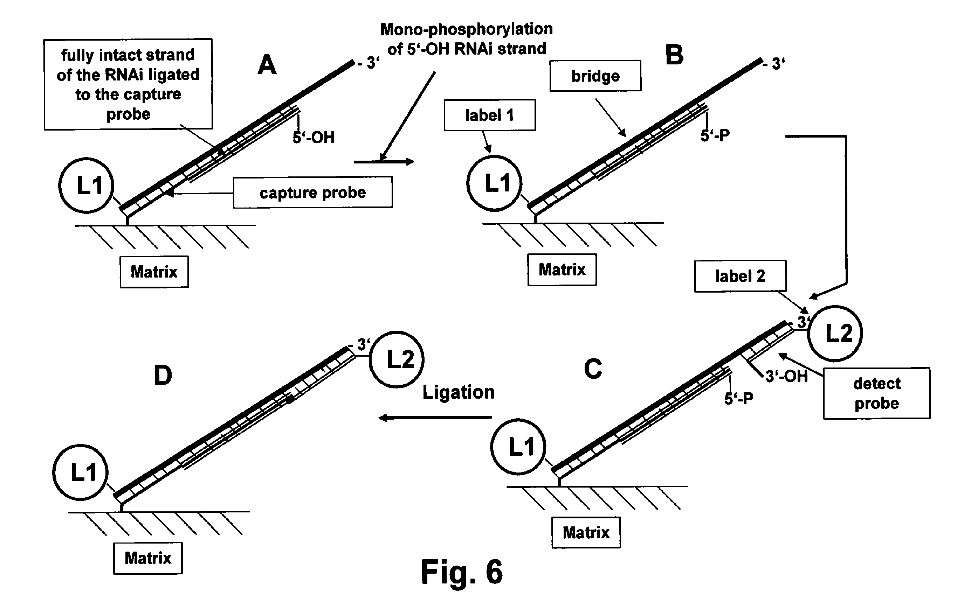

The detect probe shares a minimal number of nucleotides that allows hybridisation to the 3'- end of the bridge oligonucleotide that are used for template-directed ligation. Therefore the detect probe comprises two to 20 nucleotides, preferably 10 to 15 nucleotides (Fig. 6C). The detect probe carries a marker molecule or label that can be detected. The label can in principle be linked to each nucleotide. Preferably, the label is located at the 5'-end of the detect probe whereby between 5'-end of the detect probe and the label a linker can be inserted. The linker can be formed by hydrophilic linkers of skilled in the art or by D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides and/or L-LNA nucleotides (Fig. 6C).

The detection of RNAi molecules can be carried out as follows:
At first the capture probe is immobilised to a surface or matrix (Fig. 5A). Secondly, the bridge is added and hybridise to the capture probe forming a double-stranded oligonucleotide whereby the nucleotides of the 3'-end of the bridge are not paired to other nucleotides and are free to hybridize to one out of the two strands of the RNAi molecule to be measured (Fig. 5B). In the following the RNAi molecule that a short time before was denatured leading to two separated strands of the RNAi molecule to be measured is added (Fig. 5C). Alternatively, the denaturation can be done in presence of RNAi molecule and bridge or RNAi molecule, bridge and capture probe. A further alternative is that the double-strands of the RNAi molecule were separated from each other in advance (e.g. by a denaturation step) and one out of the two strands of the RNAi molecule were taken out (e.g. by hybridisation to a complementary oligonucleotide) and the second strand is given to the immobilised capture probe.

The one out of the two strands of the RNAi molecule to be measured hybridises to bridge (Fig. 5C). Due to the hybridisation of the strand of the RNAi molecule to bridge, the 3'-OH the strand of the RNAi molecule and the 5'-monophosphate of the capture probe are brought together and can be linked by ligation using DNA ligase (Fig. 5D). This action can only occur, if the RNAi strand is fully intact. Because of ligation reaction, a double-stranded oligonucleotide is formed by the bridge, and the ligated RNAi strand plus the capture probe. The two strands of the double-stranded oligonucleotide can be separated from each other at much more stringent wash conditions than the bridge from the capture probe, because the bridge and the to the RNAi strand ligated capture probe are forming much more base pairs than only the bridge and the capture probe. The bridge to which the RNAi strand is hybridised can be easily washed away, if at the 3'-end of the RNAi strand one nucleotide or more nucleotides are missing, and therefore no ligation reaction to the capture probe can occur. However, if no ligation reaction occured, the bridge oligonucleotide plus the defect RNAi strand can be removed by washing from the reaction batch (Fig. 5D).

At this time of the procedure, the first time the amount of the intact (3'-intact) RNAi strand can be determined. The amount of remained bridge (bound to the capture probe) can be measured and compared to a control which comprise the same amount of bridge that was present before adding the RNAi molecules. Calculating from the reduction of the signal in comparison to the control, the amount of the bound RNAi strand (and therefore RNAi at all) can be determined.

In order to determine if the 5'-end of the RNAi strand to be measured is also intact, a specific ligation reaction at its 5'-end are carried out. For that, after one or several washing steps the 5'-OH of the strand of the RNAi molecule is mono-phosphorylated (e.g. using T4 Kinase) (Fig. 5A to 5B). After one or several washing steps in order to remove reactants, the detect probe are added whereby the detect probe hybridize to the 3'-end of the bridge (Fig. 6C). Due to the hybridisation, the 5'-monophosphorylated end of the strand of the RNAi molecule and the 3'-OH of the detect probe are brought together and can be linked by ligation using DNA ligase. After several washing steps the amount of bound detect probe (carrying a label or marker molecule) can be measured. Calculating from the amount of signal the amount of the bound fully intact (5'-and 3'-ends do not miss one or more nucleotides) RNAi strand (and therefore RNAi at all) can be determined (Fig. 6D).

The label or marker molecule of the bridge and the detect probe, respectively should be different, so that the one can be detected independently from the other. Alternatively, both labels and marker molecules can be part of FRET system

The described RNAi detection assay can be done either for the first or second strand out of the two strands of the RNAi molecule. Alternatively, both strands out of the two strands of the RNAi molecule can be detected in parallel reaction batches using the respective designed capture probes, detect probes and bridges. In order to quantify the amount of the RNAi at all, the amount of both strands should be measured. The analysis should be lead to more or less equal amounts of the first and the second strand of the RNAi molecule.

### Example 6: RNAi detection assay using a double-stranded RNAi molecule

The RNAi detection assay as described in connection with example 2 cannot be used not only for one out of the two strands of the RNAi molecule, respectively, but also for a fully intact ds RNAi molecule.

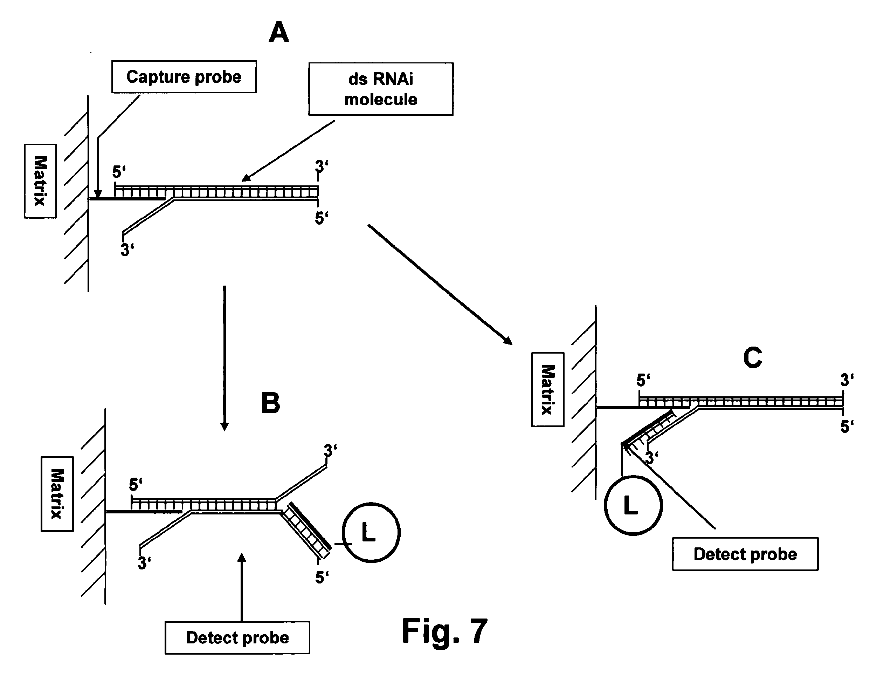

For this purpose the capture probe comprise a terminal sequence stretch that can hybridize to the 5'- or 3'-terminal end of the first strand of the RNAi molecule and the detect comprise a terminal sequence stretch that can hybridize to the 5'- or 3'-terminal end of the second strand of the RNAi molecule. Alternatively, the capture probe comprise a terminal sequence stretch that can hybridize to the 5'- or 3'-terminal end of the second strand of the RNAi molecule and the detect comprise a terminal sequence stretch that can hybridize to the 5'- or 3'-terminal end of the first strand of the RNAi molecule (Fig. 7).

### Example 7: Synthesis of siRNA molecules, Spiegelmer molecules, probe molecules, bridge molecules and derivatization of Spiegelmer molecules

### Small scale synthesis

siRNA molecules, Spiegelmers, capture and detection probes were produced by solid-phase synthesis with an ABI 394 synthesizer (Applied Biosystems, Foster City, CA, USA) using 2'TBDMS RNA or DNA phosphoramidite chemistry (M.J. Damha, K.K. Ogilvie, Methods in Molecular Biology, Vol. 20 Protocols for oligonucleotides and analogs, ed. S. Agrawal, p. 81-114, Humana Press Inc. 1993). rA(N-Bz)-, rC(Ac)-, rG(N-ibu)-, and rU-phosphoramidites or A(N-Bz)-, C(Ac)-, G(N-ibu)-, and T-phosphoramidites in the D- and L-configuration were purchased from ChemGenes, Wilmington, MA. Aptamers and Spiegelmers were purified by gel electrophoresis or by HPLC.

### Large scale synthesis plus modification

Spiegelmers mNOX-E36 (SEQ.ID. 3) and mNOX-E36-3'-PEG (SEQ.ID. 4) were produced by solid-phase synthesis with an ÄktaPilot100 synthesizer (Amersham Biosciences; General Electric Healthcare, Freiburg) using 2'TBDMS RNA phosphoramidite chemistry (M.J. Damha, K.K. Ogilvie, Methods in Molecular Biology, Vol. 20 Protocols for oligonucleotides and analogs, ed. S. Agrawal, p. 81-114, Humana Press Inc. 1993). L-rA(N-Bz)-, L-rC(Ac)-, L-rG(N-ibu)-, and L-rU- phosphoramidites were purchased from ChemGenes, Wilmington, MA. Synthesis of the unmodified Spiegelmer was started on L-riboG modified CPG pore size 1000 Å (Link Technology, Glasgow, UK); for the 3'-NH₂-modified Spiegelmer, 3'-Aminomodifier-CPG, 1000 Å (ChemGenes, Wilmington, MA) was used. For coupling (15 min per cycle), 0.3 M benzylthiotetrazole (CMS-Chemicals, Abingdon, UK) in acetonitrile, and 3.5 equivalents of the respective 0.1 M phosphoramidite solution in acetonitrile was used. An oxidation-capping cycle was used. Further standard solvents and reagents for oligonucleotide synthesis were purchased from Biosolve (Valkenswaard, NL). The Spiegelmer was synthesized DMT-ON; after deprotection, it was purified via preparative RP-HPLC (Wincott F. et al. (1995) Nucleic Acids Res 23:2677) using Source15RPC medium (Amersham). The 5'DMT-group was removed with 80% acetic acid (30 min at RT). Subsequently, aqueous 2 M NaOAc solution was added and the Spiegelmer was desalted by tangential-flow filtration using a 5 K regenerated cellulose membrane (Millipore, Bedford, MA).

### PEGylation of NOX-E36

In order to prolong the Spiegelmer's plasma residence time *in vivo,* Spiegelmer mNOX-E36 was covalently coupled to a 40 kDa polyethylene glycol (PEG) moiety at the 3'-end (leading to Spiegelmer mNOX-E36-3'-PEG).

For PEGylation (for technical details of the method for PEGylation see European patent application EP 1 306 382), the purified 3'-amino modified Spiegelmer was dissolved in a mixture of H₂O (2.5 ml), DMF (5 ml), and buffer A (5 ml; prepared by mixing citric acid H₂O [7 g], boric acid [3.54 g], phosphoric acid [2.26 ml], and 1 M NaOH [343 ml] and adding H2O to a final volume of 11; pH = 8.4 was adjusted with 1 M HCl).

The pH of the Spiegelmer solution was brought to 8.4 with 1 M NaOH. Then, 40 kDa PEG-NHS ester (Nektar Therapeutics, Huntsville, AL) was added at 37°C every 30 min in four portions of 0.6 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), 4 ml buffer A, and 4 ml buffer B (0.1 M triethylammonium acetate in H₂O and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAC. The PEGylated Spiegelmer was desalted by tangential-flow filtration (5 K regenerated cellulose membrane, Millipore, Bedford MA).

### Example 8: Detection of siRNA using RNAi detection assay based on ligation

In order to evaluate the assay as described in example 5 three different siRNA molecules were synhesized and used in said assay, whereby all siRNA molecules have the same sequence, but they consisted of D-RNA, D-phoshorothioate RNA or a combination of D-RNA and D-2'-O-Methyl-RNA.

### siRNA (D-RNA)

first strand sequence: 4x A, 6x G, 6x C, 3x U
second strand sequence: 5x G, 6x C, 4x U, 4x A

### Phosphorothioat si-RNA (D- Phosphorothioat-RNA)

first strand sequence: 4x A, 6x G, 6x C, 3x U
second strand sequence: 5x G, 6x C, 4x U, 4x A

### 2'-O-Methyl siRNA (Combination of D-RNA and D-2'-O-Methyl-RNA)

first strand sequence: 4x A, 6x G, 6x C, 3x U
second strand sequence: 5x G, 6x C, 4x U, 4x A

### siRNA Capture probe:

5'-P-GATGGCGCCACTCCGTCGTG-C18_Spacer- C18 Spacer-3'-Amino (SEQ.ID. 1)

### siRNA bridge:

5'-Biotin-C18_Spacer-GCGCCAUC-Sequence_of_the_first strand (SEQ.ID. 2)

**coupling buffer:** 0,5 M Na₂HPO₄, 1 mM EDTA, pH 8,5

**hybridisation buffer:** 0,45 M NaCL, 0,045 M Na₃-citrate, 0,5% Sodium lauroylacrosine, pH 7.0
**1xTBST:** 20 mM Tris-Cl, 137 mM Na Cl, 0,1% (v/v) Tween 20, PH 7,6

A DNA-Bind plate (Costar, white) was incubated with 0,75 µM capture probe (in coupling buffer) for 2 hours at room temperature. Unbound capture probe was removed by several washing steps with hybridisation buffer.

In PCR-plate 40 nM bridge (in hybridisation buffer) was incubated with different amounts of the 3 different siRNA molecules. The plate was heated for 5 min at 95°C and cooled down for

15 min at room temperature. The samples were transferred to the DNA-Bind-plate and incubated with immobilized capture probe for 2 hours on a shaker (500 rpm). After several washing steps with hybridisation and ligation buffer the ligation reaction was carried out for 12 hours at room temperature.

### Protocol for Ligation :

| **Component** | **final concentration** |
|---|---|
| 10 ligation buffer | 1x |
| DTT | 3 mM |
| PEG4000 | 5% |
| ATP | 0,5 mM |
| Ligase | 0,25 U/ µL |

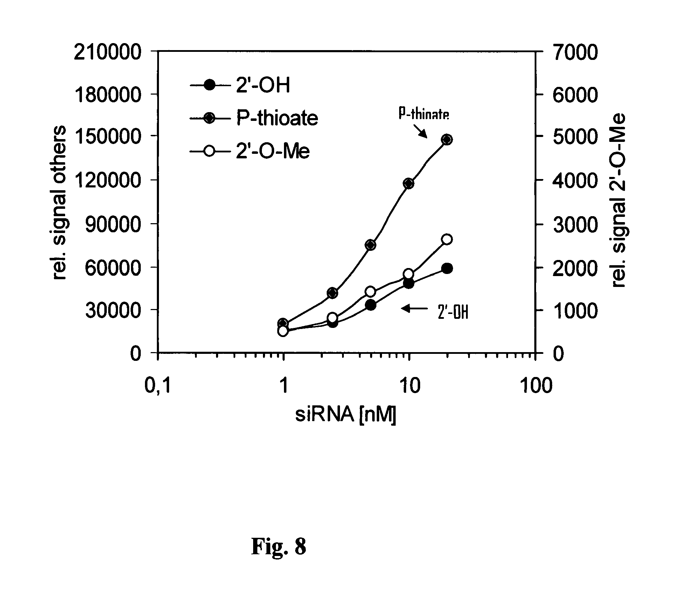

After ligation the samples were washed with 1x TBST buffer and streptavidin-alkaline phosphatase conjugate was added. The conjugation between biotin and streptavidin was carried out for 1 hour at room temperature. Then the chemiluminescence substrate was added and the signals were detected. It could be shown that all determined siRNA molecules could be detected using the ligation assay (see Fig. 8).

### Example 9: Quantification of Spiegelmer mNOX-E36 in plasma of mice

Amount of the Spiegelmers mNOX-E36 (Seq.ID. 3) and mNOX-E36-3'PEG (Seq.ID. 4) in the plasma samples of mice treated three times per week with Spiegelmers mNOX-E36 and mNOX-E36-3'PEG were quantified by a sandwich hybridisation assay based on an assay as described in example 2. Blood samples were collected in parallel to administration to follow the plasma clearance of mNOX-E36 and mNOX-E36-3'PEG . The Spiegelmers mNOX-E36 and mNOX-E36-3'PEG are described in more detail in the international patent application PCT/EP2007/001294.

### Animals and Experimental Protocol

Ten week old female MRL^{lpr/lpr} mice were obtained from Harlan Winkelmann (Borchen, Germany) and kept under normal housing conditions in a 12 hour light and dark cycle. Water and standard chow (Ssniff, Soest, Germany) were available *ad libitum.* At age 14 weeks, groups of 12 mice received subcutaneous injections of Spiegelmers in 5 % glucose (injection volume, 4 ml/kg) three times per week as follows: mNOX-E36, 1.5 µmol/kg; mNOX-E36-3'PEG. The plasma levels of mNOX-E36 and mNOX-E36-3'PEG were determined from blood samples taken weekly from the retroorbital sinus 3 or 24 hours after injection, respectively. Mice were sacrificed by cervical dislocation at the end of week 24 of age.

### Assay Protocol

### Hybridisation plate preparation

The mNOX-E36 capture probe (Seq.ID. 5) that is useable for both Spiegelmers, mNOX-E36 and mNOX-E36-3'PEG, was immobilized to white DNA-BIND 96well plates (Corning Costar, Wiesbaden, Germany) at 0.75 mM in 0.5 M sodium phosphate, 1 mM EDTA, pH 8.5 over night at 4°C. Wells were washed twice and blocked with 0.5% w/v BSA in 0.25 M sodium phosphate, 1 mM EDTA, pH 8.5 for 3 h at 37°C, washed again and stored at 4°C until use. Prior to hybridisation, wells were pre-warmed to 37°C and washed twice with pre-warmed wash buffer (3xSSC, 0.5% [w/v] sodium dodecyl sarcosinate, pH 7.0; in advance a 20x stock [3 M NaCl, 0,3 M Na₃Citrate) is prepared without sodium lauroylsarcosine and diluted accordingly).

### Sample preparation

All samples were assayed in duplicates. Plasma samples were thawed on ice, vortexed and spun down briefly in a cooled tabletop centrifuge. Tissue homogenates were thawed at RT and centrifuged 5 min at maximum speed and RT. Only 5 µl each sample were removed for the assay, and afterwards returned to the freezer for storage. Samples were diluted with hybridisation buffer (8 nM mNOX-E36 detection probe [Seq.ID. 6] in wash buffer, mNOX-E36 detection probe is useable for both Spiegelmers, mNOX-E36 and mNOX-E36-3'PEG) at RT according to the following scheme:

| | | |
|---|---|---|
| 1:30 | 5 µl sample | + 145 µl hybridisation buffer |
| 1:300 | 20 µl 1:30 | + 180 µl hybridisation buffer |
| 1:3000 | 20 µl 1:300 | + 180 µl hybridisation buffer |
| 1:30000 | 20 µl 1:3000 | + 180 µl hybridisation buffer |

All sample dilutions were assayed. mNOX-E36 or standard mNOX-E36-3'PEG was serial diluted to a 8-point calibration curve spanning the 0-4 nM range. No QC samples were prepared and assayed. Calibration standard was identical to that of the in-study samples.

### Hybridisation and detection

Samples were heated for 10 min at 95°C and cooled to 37°C. Spiegelmer/detection probe complexes were annealed to immobilized capture probes for 30 min at 37°C. Unbound spiegelmers were removed by washing twice with wash buffer and 1 × TBST (20 mM Tris-Cl, 137 mM NaCl, 0.1% Tween 20, pH 7.5), respectively. Hybridized complexes were detected by streptavidin alkaline phosphatase diluted 1:5000 in 1× TBST for 1 h at room temperature. To remove unbound conjugate, wells were washed again with 1× TBST and 20 mM Tris-Cl, 1 mM MgCl2, pH 9.8 (twice each). Wells were fmally filled with 100 ml CSDP substrate (Applied Biosystems, Darmstadt, Germany) and incubated for 45 min at room temperature. Chemiluminescence was measured on a FLUOstar Optima microplate reader (BMG Labtechnologies, Offenburg, Germany).

### Data analysis

The following assayed sample dilutions were used for quantitative data analysis:

| | |
|---|---|
| rat EDTA plasma | 1:2000 |

The data obatained from the vehicle group (no Spiegelmer was adminstered) was subtracted as background signal.

The sandwich hybridisation assay as described herein also works in similar fashion for Spiegelmer NOX-36 (Seq.ID. 7), NOX-E36-5'-PEG (Seq.ID. 8) and NOX-E36-3'-PEG (Seq.ID. 9) whereby the respective NOX-E36 capture probe (Seq.ID. 10) and the respective NOX-E36 detection probe (Seq.ID. 11) has to be used (data not shown). The Spiegelmers are described in more detail in the international patent application PCT/EP2007/001294.

The sandwich hybridisation assay as described herein also works in similar fashion for Spiegelmer NOX-A12 (Seq.ID. 12), NOX-A12-5'-PEG (Seq.ID. 13) and NOX-A12-3'-PEG (Seq.ID. 14) whereby the respective NOX-A12 capture probe (Seq.ID. 15) and the respective NOX-A12 detection probe (Seq.ID. 16) has to be used (data not shown). The Spiegelmers are described in more detail in the international patent application PCT/EP2007/006387.

### Results

### Plasma levels of mNOX-E36 and mNOX-E36-3'PEG in MRL^{lpr/}^{lpr} mice

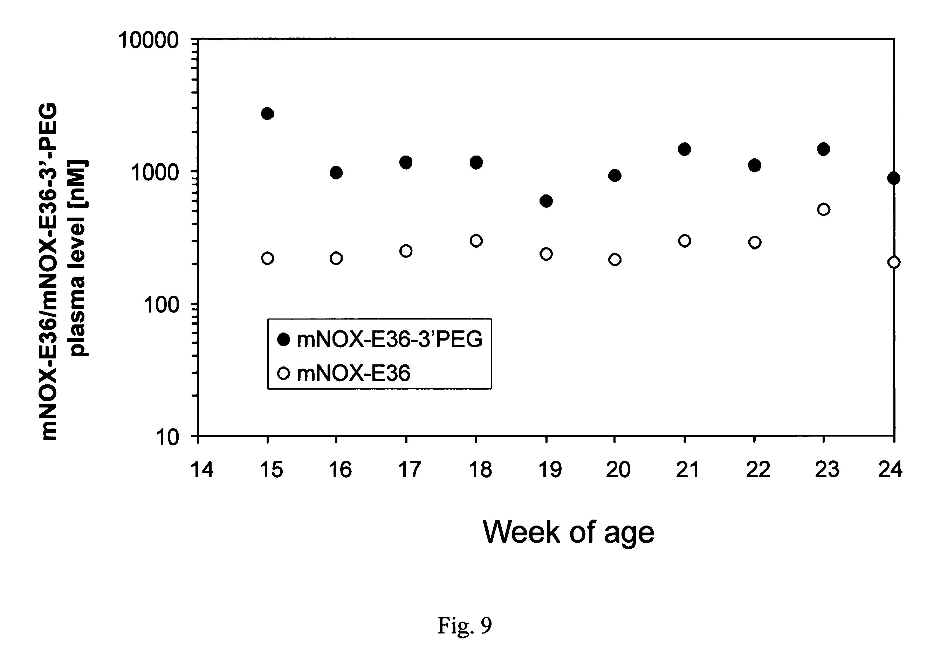

mNOX-E36 and mNOX-E36-3'PEG plasma levels were determined at weekly intervals in order to monitor drug exposure during progressive kidney disease of MRL^{lpr/lpr} mice. The median plasma levels of mNOX-E36 3 h after injection and mNOX-E36-3'PEG 24 h after injection were approximately 300 nM and 1 µM throughout the study, respectively (Fig. 9). Thus, pegylation increased the plasma levels of mNOX-E36 and the progressive kidney disease of MRL^{lpr/lpr} mice did not modulate the pharmacokinetics of both Spiegelmers.

### Embodiments

**Embodiment 1:** A method for the detection of a nucleic acid molecule comprising at least a strand comprising a sequence of nucleotides in a sample, whereby the method comprises the following steps:
   e) providing a sample containing the nucleic acid molecule;
   f) providing a capture probe, whereby the capture probe is at least partially complementary to a part of the nucleic acid molecule;
   g) allowing the capture probe to react with the nucleic acid molecule or a part thereof; and
   d) detecting whether or not the capture probe is hybridized to the nucleic acid molecule or part thereof.
**Embodiment 2:** The method according to embodiment 1, wherein in step c) a complex is formed consisting of the nucleic acid molecule and the capture probe.
**Embodiment 3:** The method according to any of embodiments 1 or 2, wherein the capture probe comprises a detection means which allows the detection of the capture probe.
**Embodiment 4:** The method according to embodiment 3, wherein after step c) a nuclease activity is added to the reaction, whereby the nuclease activity is degrading the capture probe which is not hybridized to the nucleic acid molecule.
**Embodiment 5:** The method according to any of embodiments 1 to 4, wherein the capture probe is a single- stranded nucleic acid, preferably a single-stranded ribonucleic acid, and the nuclease activity is a single-stranded specific RNAse.
**Embodiment 6:** The method according to any of embodiments 1 to 5, wherein the nucleic acid molecule is hybridizing to the capture probe over the entire length of said a strand of the nucleic acid molecule.
**Embodiment 7:** The method according to embodiment 6, wherein the complex consisting of the capture probe and said a strand of the nucleic acid molecule forms a blunt end or a protruding 3' and/ or 5'-end of the capture probe.
**Embodiment 8:** The method according to any of embodiments 1 to 7, wherein the capture probe is immobilized to a support, preferably a solid support, by the 5' end of the capture probe.
**Embodiment 9:** The method according to any of embodiments 1 to 7, wherein the capture probe is immobilized to a support, preferably a solid support, by the 3' end of the capture probe.
**Embodiment 10:** The method according to any of embodiments 1 to 9, wherein the nucleic acid molecule is a single-stranded nucleic acid molecule.
**Embodiment 11:** The method according to embodiment 10, wherein the single-stranded nucleic acid molecule consists of RNA, DNA, modified RNA, modified DNA, LNA or PNA, or combinations thereof, preferably DNA, RNA, modified RNA or modified DNA.
**Embodiment 12:** The method according to any of embodiments 1 to 9, wherein the nucleic acid molecule is a double-stranded nucleic acid molecule comprising a first strand and a second strand, and wherein the strand of the nucleic acid molecule according any of embodiments 1 to 9 is the first strand or the second strand of the double-stranded nucleic acid, or a part thereof.
**Embodiment 13:** The method according to embodiment 12, wherein the first strand or the second strand of the double-stranded nucleic acid molecule consists each and independently from each other of RNA, DNA, modified RNA or modified DNA, or combinations thereof.
**Embodiment 14:** The method according to any of embodiments 12 and 13, wherein prior, during or after step a) but before step c) the double-stranded molecule is separated into a first strand and a second strand.
**Embodiment 15:** The method according to any of embodiments 12, 13 and 14, wherein in step d) it is detected whether the capture probe is hybridized to the first strand or the second strand.
**Embodiment 16:** The method according to embodiment 1, wherein the capture probe comprises a first capture probe and a second capture probe, whereby the first capture probe is at least partially complementary to a first part of said a strand of the nucleic acid molecule and the second capture probe is at least partially complementary to a second part of said a strand of the nucleic acid molecule.
**Embodiment 17:** The method according to embodiment 16, wherein in step c) a complex is formed consisting of said a strand of the nucleic acid molecule and the first and the second capture probe.
**Embodiment 18:** The method according to any of embodiments 16 and 17, wherein either the first capture probe or the second capture probe comprises a detection means, and whereby either the second capture probe or the first capture probe can be immobilized to a support.
**Embodiment 19:** The method according to embodiment 18, wherein the first or the second capture probe is immobilized to a support, preferably a solid support, by the 5' end of the first or second capture probe.
**Embodiment 20:** The method according to embodiment 17, wherein the first or second capture probe is immobilized to a support, preferably a solid support, by the 3' end of the first or second capture probe.
**Embodiment 21:** The method according to any of the preceding embodiments, wherein the first capture probe comprises the detection means and the second capture probe is immobilized to the surface and the molecules of the first capture probe that are not part of the complex are removed from the reaction so that in step d) only the molecules of the first capture probe that are part of the complex are detected
   or
   wherein the second capture probe comprises the detection means and the first capture probe is immobilized to the surface and the molecules of the second capture probe that are not part of the complex are removed from the reaction so that in step d) only the molecules of the second capture probe that are part of the complex are detected.
**Embodiment 22:** The method according to any of embodiments 16 to 21, wherein the nucleic acid molecule is a single-stranded nucleic acid molecule.
**Embodiment 23:** The method according to embodiment 22, wherein the single-stranded molecule consists of RNA, DNA, modified RNA, modified DNA, LNA or PNA, or combinations thereof, preferably DNA, RNA, modified RNA or modified DNA.
**Embodiment 24:** The method according to embodiment 23, wherein the single-stranded molecule consists of L-RNA or L-DNA, modified L-RNA, modified L-DNA or L-LNA, or combinations thereof, preferably L-DNA and L-RNA.
**Embodiment 25:** The method according to any of embodiments 16 to 21, wherein the nucleic acid molecule is a double-stranded nucleic acid comprising a first strand and a second strand, and wherein the strand of the nucleic acid molecule according to any of embodiments 16 to 21 is the first strand or the second strand of the double-stranded nucleic acid, or a part thereof.
**Embodiment 26:** The method according to embodiment 25, wherein the first strand or the second strand of the double-stranded nucleic acid consists each and independently from each other of RNA, DNA, modified RNA, modified DNA, or combinations thereof.
**Embodiment 27:** The method according to any of embodiments 25 to 26, wherein prior, during or after step a) but before step c) the double-stranded molecule is separated into a first strand and a second strand.
**Embodiment 28:** The method according to any of embodiments 25 to 27, wherein in step d) it is detected whether the capture probe is hybridized to the first strand or the second strand.
**Embodiment 29:** A method for the detection of a nucleic acid molecule comprising at least a strand comprising a sequence of nucleotides in a sample, whereby the method comprises the following steps:
   a) providing a sample containing the nucleic acid molecule;
   b) providing a capture probe, whereby the capture probe is at least partially complementary to a part of the nucleic acid molecule;
   c) providing a detection probe comprising a detection means, whereby the detection probe is at least partially complementary to the capture probe;
   h) allowing the capture probe and the detection probe to react either simultaneously or in any order sequentially with the nucleic acid molecule or a part thereof; and
   e) detecting whether or not the capture probe is hybridized to the nucleic acid molecule.
**Embodiment 30:** The method according to embodiment 29, wherein the nucleic acid molecule comprises a nucleotide sequence which is at least partially complementary to the capture probe.
**Embodiment 31:** The method according to embodiment 30, wherein in step e) it is detected whether the capture probe is hybridized to the nucleotide sequence of the nucleic acid molecule which is complementary to the capture probe.
**Embodiment 32:** The method according to any of embodiments 29 to 31, wherein step e) comprises the step of comparing the signal generated by the detection means when the capture probe and the detecting probe are hybridized in the presence of the nucleic acid molecule or part thereof, and in the absence of the nucleic acid molecule or part thereof.
**Embodiment 33:** The method according to any of embodiments 29 to 32, wherein the nucleic acid molecule is a single-stranded nucleic acid molecule.
**Embodiment 34:** The method according to embodiment 33, wherein the single-stranded molecule consists of RNA, DNA, modified RNA, modified DNA, LNA or PNA, or combinations thereof, preferably DNA, RNA, modified RNA or modified DNA.
**Embodiment 35:** The method according to embodiment 33, wherein the single-stranded molecule consists of L-RNA or L-DNA, modified L-RNA, modified L-DNA or L-LNA, or combinations thereof, preferably L-DNA and L-RNA.
**Embodiment 36:** The method according to any of embodiments 29 to 32, wherein the nucleic acid molecule is a double-stranded nucleic acid comprising a first strand and a second strand, and wherein the strand of the nucleic acid molecule according to any of embodiments 29 to 32 is the first strand or the second strand of the double-stranded nucleic acid, or a part thereof.
**Embodiment 37:** The method according to embodiment 36, wherein the first strand or the second strand of the double-stranded nucleic acid consists each and independently from each other of RNA, DNA, modified RNA, modified DNA, or combinations thereof.
**Embodiment 38:** The method according to any of embodiments 36 to 37, wherein prior, during or after step a) but before step c) the double-stranded molecule is separated into a first strand and a second strand.
**Embodiment 39:** A method for the detection of a nucleic acid molecule comprising at least a strand comprising a sequence of nucleotides in a sample, whereby the method comprises the following steps:
   h) providing a sample containing the nucleic acid molecule;
      whereby a strand of the nucleic acid molecule consists of a first part and a second part;
   i) providing a capture probe;
   j) providing a first bridging probe, whereby a first part of the first bridging probe is at least partially complementary to the capture probe and a second part of the first bridging probe is at least partially complementary to the first part of said a strand of the nucleic acid and whereby when the second part of the bridging probe is hybridized to the first part of said a strand of the nucleic acid, the second part of the strand forms an overhang;
   k) providing a second bridging probe comprising a first part and a second part, whereby the first part of the second bridging probe is at least partially complementary to the second part of said a strand of the nucleic acid, and allowing the second bridging probe to hybridise to the overhang formed in step c);
   l) providing a detection probe, whereby the detection probe is at least partially complementary to the second part of the second bridging probe and comprises a detection means, and allowing the detection probe to hybridize to the second part of the second bridging probe;
   m) ligating one end of said a strand of the nucleic acid to one end of the detection probe and/or the other end of the respective strand to one end of the capture probe; and
   n) detecting whether or not the capture probe and/or the detection probe is/are ligated to said a strand of the nucleic acid provided in step a).
**Embodiment 40:** The method according to embodiment 39, wherein the ligation between the terminal nucleotide of the capture probe and the terminal nucleotide of said a first part of the strand of the nucleic acid molecule occurs under the proviso that the terminal nucleotide of the capture probe and the terminal nucleotide of the first part of said a strand of the nucleic acid molecule are immediately adjacent to each other.
**Embodiment 41:** The method according to any of embodiments 39 and 40, wherein the capture probe and the first part of said a strand of the nucleic acid molecule, taken together, form a nucleotide sequence which has about the same length and/or which comprises a nucleotide sequence which is essentially complementary to the first bridging probe or an part thereof consisting of adjacent nucleotides.
**Embodiment 42:** The method according to any of embodiments 39 to 40, wherein the ligation between the terminal nucleotide of the detection probe and the terminal nucleotide of the second part of said a strand of the nucleic acid molecule occurs under the proviso that the terminal nucleotide of the detection probe and the terminal nucleotide of the second part of said a strand of the nucleic acid molecule are immediately adjacent to each other.
**Embodiment 43:** The method according to any of embodiments 39 to 42, wherein the detection probe and the second part of said a strand of the nucleic acid molecule, taken together, form a nucleotide sequence which has at least the sequence of the second bridging probe and/or which comprises a nucleotide sequence which is essentially complementary to the second bridging probe or a part thereof consisting of adjacent nucleotides.
**Embodiment 44:** The method according to any of embodiments 39 to 40, wherein, after the ligation, the detection probe not ligated to said a strand of the nucleic acid molecule and/or said a strand of the nucleic acid molecule not ligated to the capture probe is/are removed from the reaction.
**Embodiment 45:** The method according to any of embodiments 39 to 44, wherein the capture probe is immobilized to a support, preferably a solid support, by the 3' end of the capture probe.
**Embodiment 46:** The method according to embodiment 45, wherein the capture probe provides for a 5' end and/or said a strand of the nucleic acid molecule immobilized to the first bridging probe provides for a 5' end, whereby one or both of the 5'ends are monophosphorylated.
**Embodiment 47:** The method according to any of embodiments 39 to 44, wherein the capture probe is immobilized to a support, preferably a solid support, by the 5' end of the capture probe.
**Embodiment 48:** The method according to embodiment 47, wherein the detection probe provides for a 5' end and/or said a strand of the nucleic acid molecule immobilized to the first bridging probe provides for a 5' end, whereby the one or both of the 5'ends are monophosphorylated.
**Embodiment 49:** The method according to any of embodiment 39 to 48, wherein the first bridging probe and the second bridging probe are a single molecule.
**Embodiment 50:** The method according to embodiment 49, wherein the single molecule is created prior or subsequently to the addition of both the first bridging probe and the second bridging probe to the reaction, preferably by a ligase activity.
**Embodiment 51:** The method according to any of embodiments 39 to 50, wherein the stretch of nucleotides of the capture probe interacting with the first bridging probe has a length of about 2 to 20 consecutive nucleotides, preferably 6 to 15 consecutive nucleotides.
**Embodiment 52:** The method according to any of embodiments 39 to 50, wherein the first part of the first bridging probe has a length of about 2 to 20 consecutive nucleotides, preferably 6 to 15 consecutive nucleotides.
**Embodiment 53:** The method according to any of embodiments 39 to 52, wherein the second part of the first bridging probe has a length of about 2 to 20 consecutive nucleotides, preferably 7 to 11 consecutive nucleotides.
**Embodiment 54:** The method according to any of embodiments 39 to 53, wherein the detection probe comprises a length of about 2 to 20 consecutive nucleotides, preferably about 7 to 11 consecutive nucleotides.
**Embodiment 55:** The method according to any of embodiments 39 to 54, wherein the nucleic acid molecule is a single-stranded nucleic acid molecule.
**Embodiment 56:** The method according to embodiment 55, wherein the single-stranded molecule consists of RNA, DNA, modified RNA or modified DNA, preferably DNA, RNA, modified RNA or modified DNA.
**Embodiment 57:** The method according to any of embodiments 39 to 54, wherein the nucleic acid molecule is a double-stranded nucleic acid molecule comprising a first strand and a second strand, and wherein the strand of the nucleic acid molecule according to any of embodiments 39 to 54 is the first strand or the second strand of the double-stranded nucleic acid, or a part thereof.
**Embodiment 58:** The method according to embodiment 57, wherein the first strand or the second strand of the double-stranded nucleic acid consists each and independently from each other of RNA, DNA, modified RNA, modified DNA, or combinations thereof.
**Embodiment 59:** The method according to any of embodiments 57 and 58, wherein prior, during or after step a) but before step c) the double-stranded molecule is separated into a first strand and a second strand.
**Embodiment 60:** The method according to any of embodiments 57 to 59, wherein in step e) it is detected whether the capture probe is hybridized to the first strand or the second strand.
**Embodiment 61:** A method for the detection in a sample of a nucleic acid molecule comprising at least a strand comprising a sequence of nucleotides, whereby the method comprises the following steps:
   e) providing a sample containing the nucleic acid molecule;
   f) providing a capture probe;
   g) providing a bridging probe comprising a detection means, whereby a first part of the bridging probe is at least partially complementary to the capture probe, a second part of the bridging probe is at least partially complementary to said a strand of the nucleic acid molecule and a third part of the bridging probe is at least partially complementary to a detection probe, and whereby when the second part of the bridging probe is hybridized to said a strand of the nucleic acid, the third part of the bridging probe forms an overhang;
   h) ligating one end of either the said a strand of the nucleic acid molecule to one end of the capture probe; and
   detecting whether or not the bridging probe is hybridized to said a strand of the nucleic acid molecule ligated to the capture probe.
**Embodiment 62:** The method according to embodiment 61, wherein either the capture probe or said a strand of the nucleic acid molecule provides for the monophosphorylated 5' end required for the ligation in step d).
**Embodiment 63:** The method according to any of embodiments 61 to 62, wherein the capture probe and said a strand of the nucleic acid molecule, taken together, form a nucleotide sequence which is essentially complementary to a stretch of consecutive nucleotides comprising the second part of the bridging probe and at least a part of the first part of the bridging probe.
**Embodiment 64:** The method according to any of embodiments 61 to 63, wherein the capture probe provides for a monophosphorylated 5' end and the 3'end of said a strand of the nucleic acid molecule is ligated to said monophosphorylated 5' end of the capture probe, whereas a strand of the nucleic acid molecule the 3' end of which differs in the nucleotide sequence from the 3' end of said a strand of the nucleic acid molecule at the 3' end of one or more nucleotides will not be ligated.
**Embodiment 65:** The method according to any of embodiments 61 to 64, wherein the method further comprises the step of:
   e) providing a detection probe, whereby the detection probe comprises a detection means which is different from the detection means of the bridging probe, and whereby the detection probe is at least partially complementary to the third part of the bridging probe;
   f) ligating one end of said a strand of the nucleic acid to one end of the detection probe; and
   g) detecting whether or not the detection probe is ligated to said a strand of the nucleic acid molecule.
**Embodiment 66:** The method according to embodiment 65, wherein either the detection probe or said a strand of the nucleic acid molecule provides for the monophosphorylated 5' end required for the ligation in step g).
**Embodiment 67:** The method according to any of embodiments 65 to 66, wherein the detection probe and said a strand of the nucleic acid molecule, taken together, form a nucleotide sequence which is essentially complementary to a stretch of consecutive nucleotides comprising the second part of the bridging probe and at least a part of the third part of the bridging probe.
**Embodiment 68:** The method according to any of embodiments 65 to 67, wherein the detection probe provides for a 3' end and the 5'end of said a strand of the nucleic acid molecule provided for a monophosphorylated 5' end which is ligated to said 3' end of the detection probe, whereas a strand which differs from the 5' end of said a strand of the nucleic acid molecule at the 5' end of one or more nucleotides will not be ligated.
**Embodiment 69:** The method according to any of embodiments 65 to 68, wherein the detection means of the capture probe and the detection means of the detection probe form a FRET system or are at least a part thereof.
**Embodiment 70:** The method according to any of embodiments 61 to 69, wherein the length of the first part of the bridging probe is about 2 to 15 consecutive nucleotides, preferably 6 to 10 consecutive nucleotides.
**Embodiment 71:** The method according to any of embodiments 61 to 70, wherein the length of the second part of the bridging probe is essentially identical to the length of said a strand of the nucleic acid molecule.
**Embodiment 72:** The method according to any of embodiments 61 to 71, wherein the length of the third part of the bridging probe is about 2 to 20 consecutive nucleotides, preferably 10 to 15 nucleotides.
**Embodiment 73:** The method according to any of embodiments 61 to 72, wherein the bridging probe consist of a first molecule and a second molecule, whereby the first molecule is a single-stranded nucleic acid molecule comprising the first part of the bridging probe and the second part of the bridging probe, and the second molecule is a single-stranded nucleic acid molecule comprising the third part to the bridging probe.
**Embodiment 74:** The method according to any of embodiments 61 to 73, wherein the nucleic acid molecule is a single-stranded nucleic acid molecule.
**Embodiment 75:** The method according to embodiment 74, wherein the single-stranded molecule consists of RNA, DNA, modified RNA or modified DNA, preferably DNA, RNA, modified RNA or modified DNA.
**Embodiment 76:** The method according to any of embodiments 61 to 73, wherein the nucleic acid molecule is a double-stranded nucleic acid comprising a first strand and a second strand, and wherein the strand of the nucleic acid molecule according to any of embodiments 61 to 73 is the first strand or the second strand of the double-stranded nucleic acid, or a part thereof.
**Embodiment 77:** The method according to embodiment 76, wherein the first strand or the second strand of the double-stranded nucleic acid consists each and independently from each other of RNA, DNA, modified RNA, modified DNA, or combinations thereof.
**Embodiment 78:** The method according to any of embodiments 76 to 77, wherein prior, during or after step a) but before step c) the double-stranded molecule is separated into a first strand and a second strand.
**Embodiment 79:** The method according to any of embodiments 76 to 78, wherein in step d) it is detected whether the capture probe is hybridized to the first strand or the second strand.
**Embodiment 80:** A method for the detection in a sample of a double-stranded nucleic acid molecule comprising a first strand and a second strand, whereby the method comprises the following steps:
   a) providing a sample containing the double-stranded nucleic acid molecule;
   b) providing a capture probe, whereby the capture probe is at least partially complementary to a part of either the first strand or the second strand of the double-stranded nucleic acid molecule and is immobilzable to a support,
   c) providing a detection probe, whereby the detection probe is at least partially complementary to a part of either the second strand or the first strand of the double-stranded nucleic acid molecule and provides for a detection means;
   d) allowing the sample and more specifically the double-stranded nucleic acid molecule contained therein, the capture probe and the detection probe to react; and
   e) detecting whether the detection probe is attached to the double-stranded nucleic acid molecule and the capture probe is attached to the double-stranded nucleic acid molecule.
**Embodiment 81:** The method according to embodiment 80, wherein the attachement of step e) is a hybridization.
**Embodiment 82:** The method according to any of embodiments 80 to 81, wherein, in step e) or d), the detection probe is attached to a part of the first strand of the double-stranded nucleic acid and the capture probe is attached to a part of the second strand of the double-stranded nucleic acid, or the detection probe is attached to a part of the second strand of the double-stranded nucleic acid and the capture probe is attached to a part of the first strand of the double-stranded nucleic acid.
**Embodiment 83:** The method according to any of embodiments 80 to 82, wherein the double-stranded nucleic acid molecule is present, at least in step d) and/or step e) as a double-stranded nucleic acid molecule.
**Embodiment 84:** The method according to any of embodiments 80 to 83, wherein the detection probe and the capture probe are attached to the same end of the double-stranded nucleic acid molecule, whereby such end is defined by the 5' end of one strand and the 3' end of the other strand of the double-stranded nucleic acid molecule.
**Embodiment 85:** The method according to any of embodiments 80 to 84, wherein the detection probe and the capture probe are attached at different ends of the double-stranded nucleic acid molecule, whereby such ends are defined by the 5' end of the first strand and the 3' end of the second strand, and by the 3' end of the first strand and the 5' end of the second strand, respectively.
**Embodiment 86:** The method according to any of embodiments 1 to 85, wherein the detection means is selected from the group comprising tags and labels.
**Embodiment 87:** The method according embodiment 86, wherein the tag is selected from the group comprising biotin, streptavidin, avidin, nucleic acids, polypeptides and proteins.
**Embodiment 88:** The method according to any of embodiments 1 to 87, wherein the tag is either directly or through a linker attached to a nucleotide, preferably the 5' terminal nucleotide.
**Embodiment 89:** The method according to embodiment 86, wherein the label is selected from the group comprising radioactive labels, enzymatic labels, fluorescent labels, Cy-3, Cy-5, molecular beacons, bromo-desoxyuridine labels, a digoxigenin labels and chelator molecules.
**Embodiment 90:** The method according to embodiment 89, wherein the label is either directly or through a linker attached to a nucleotide, preferably the 5' terminal nucleotide.
**Embodiment 91:** The method according to any of embodiments 1 to 90, wherein any of the probes is a single-stranded nucleic acid molecule.
**Embodiment 92:** The method according to any of embodiments 1 to 91, wherein the nucleic acid molecule is a D-nucleic acid molecule or PNA based nucleic acid molecule and any of the capture probes, detection probes and bridging probes, each and independently, consists of nucleotides and derivatives, whereby such nucleotides and derivatives, respectively, are preferably selected from the group comprising D-deoxribonucleotides, modified D-deoxyribonucleotides, D-ribonucleotides, modified D-ribonucleotides, D-LNA nucleotides, PNA nucleotides, and any combination thereof.
**Embodiment 93:** The method according to any of the preceding embodiments wherein the nucleic acid molecule is a L-nucleic acid molecule and any of the capture probes, detection probes and bridging probes, each and independently, consists of nucleotides and derivatives, whereby such nucleotides and derivatives, respectively, are preferably selected from the group comprising L-deoxribonucleotides, modified L-deoxyribonucleotides, L-ribonucleotides, modified L-ribonucleotides, L-LNA nucleotides, and any combination thereof.
**Embodiment 94:** The method according to any of embodiments 1 to 93, wherein the capture probe and/or any bridging probe is attached to the support either directly or through a linker.
**Embodiment 95:** The method according to any of embodiments 88, 90 and 94, wherein the linker is selected from the group comprising hydrophilic linker, D-DNA nucleotides, modified D-DNA nucleotides, D-RNA nucleotides, modified D-RNA nucleotides, D-LNA nucleotides, PNA nucleotides, L-RNA nucleotides, L-DNA nucleotides, modified L-RNA nucleotides, modified L-DNA nucleotides L-LNA nucleotides, and any combination thereof.

The features of the present invention disclosed in the specification, the claims and/or the drawings may both separately and in any combination thereof be material for realizing the invention in various forms thereof.

## Claims

1. A method for the detection of a nucleic acid molecule comprising at least a strand comprising a sequence of nucleotides in a sample, whereby the method comprises the following steps:
a) providing a sample containing the nucleic acid molecule;
b) providing a capture probe, whereby the capture probe is at least partially complementary to a part of the nucleic acid molecule;
c) allowing the capture probe to react with the nucleic acid molecule or a part thereof; and
d) detecting whether or not the capture probe is hybridized to the nucleic acid molecule or part thereof,
wherein the capture probe comprises a first capture probe and a second capture probe, whereby the first capture probe is at least partially complementary to a first part of said a strand of the nucleic acid molecule and the second capture probe is at least partially complementary to a second part of said a strand of the nucleic acid molecule.

2. The method according to claim 1, wherein in step c) a complex is formed consisting of said a strand of the nucleic acid molecule and the first and the second capture probe.

3. The method according to any of claims 1 and 2, wherein either the first capture probe or the second capture probe comprises a detection means, and whereby either the second capture probe or the first capture probe can be immobilized to a support.

4. The method according to claim 3, wherein the first or the second capture probe is immobilized to a support, preferably a solid support, by the 5' end of the first or second capture probe.

5. The method according to claim 3, wherein the first or second capture probe is immobilized to a support, preferably a solid support, by the 3' end of the first or second capture probe.

6. The method according to any of claims 3 to 5, wherein the first capture probe comprises a detection means and the second capture probe is immobilized to the surface and the molecules of the first capture probe that are not part of the complex are removed from the reaction so that in step d) only the molecules of the first capture probe that are part of the complex are detected
or
wherein the second capture probe comprises a detection means and the first capture probe is immobilized to the surface and the molecules of the second capture probe that are not part of the complex are removed from the reaction so that in step d) only the molecules of the second capture probe that are part of the complex are detected.

7. The method according to any of claims 1 to 6, wherein the nucleic acid molecule is a single-stranded nucleic acid molecule.

8. The method according to claim 7, wherein the single-stranded molecule consists of RNA, DNA, modified RNA, modified DNA, LNA or PNA, or combinations thereof, preferably DNA, RNA, modified RNA or modified DNA.

9. The method according to claim 8, wherein the single-stranded molecule consists of L-RNA or L-DNA, modified L-RNA, modified L-DNA or L-LNA, or combinations thereof, preferably L-DNA and L-RNA.

10. The method according to any of claims 1 to 6, wherein the nucleic acid molecule is a double-stranded nucleic acid comprising a first strand and a second strand, and wherein the strand of the nucleic acid molecule according to any of claims 1 to 6 is the first strand or the second strand of the double-stranded nucleic acid, or a part thereof.

11. The method according to claim 10, wherein the first strand or the second strand of the double-stranded nucleic acid consists each and independently from each other of RNA, DNA, modified RNA, modified DNA, or combinations thereof.

12. The method according to any of claims 10 to 12, wherein prior, during or after step a) but before step c) the double-stranded molecule is separated into a first strand and a second strand.

13. The method according to any of claims 10 to 12, wherein in step d) it is detected whether the capture probe is hybridized to the first strand or the second strand.


SEQUENCE LISTING

<110>  NOXXON Pharma AG

<120>  Methods for the detection of a specific double-stranded nucleic
acid molecule

<130>  N 10054 PCT-EP/D-I

<150>  EP 06 022 735.2
<151>  2006-10-31

<160>  16

<170>  PatentIn version 3.1

<210>  1
<211>  20
<212>  DNA
<213>  Artificial sequence

<220>
<223>  Chemically synthesised

<220>
<221>  misc_feature
<222>  (1)..(1)
<223>  -P-


<220>
<221>  misc_feature
<222>  (20)..(20)
<223>  -(Spacer18)2-3'-NH2


<400>  1
gatggcgcca ctccgtcgtg                                                20


<210>  2
<211>  8
<212>  RNA
<213>  Artificial sequence

<220>
<223>  Chemically synthesised

<220>
<221>  misc_feature
<222>  (1)..(8)
<223>  L-RNA


<220>
<221>  misc_feature
<222>  (1)..(1)
<223>  5'-Biotin-C18_Spacer-

<220>
<221> misc_feature
<222> (8)..(8)
<223> -Sequence_of_the_first_siRNA_strand

<400> 2
gcgccauc 8

<210> 3
<211> 50
<212> RNA
<213> Artificial sequence

<220>
<223> Chemically synthesised

<220>
<221> misc_feature
<222> (1)..(50)
<223> L-RNA

<400> 3
ggcgacauug guugggcaug aggcgaggcc cuuugaugaa uccgcggcca 50

<210> 4
<211> 50
<212> RNA
<213> Artificial sequence

<220>
<223> Chemically synthesised

<220>
<221> misc_feature
<222> (1)..(50)
<223> L-RNA

<220>
<221> misc_feature
<222> (50)..(50)
<223> 40 kDa-PEG

<400> 4
ggcgacauug guugggcaug aggcgaggcc cuuugaugaa uccgcggcca 50

<210> 5
<211> 11
<212> DNA
<213> Artificial sequence

<220>
<223> Chemically synthesised

<220>
<221> misc_feature
<222> (11)..(11)
<223> -(Spacer18)2-NH4+ -3'

<220>
<221> misc_feature
<222> (1)..(11)
<223> L-DNA

<400> 5
ccaatgtcgc c 11

<210> 6
<211> 9
<212> RNA
<213> Artificial sequence

<220>
<223> Chemically synthesised

<220>
<221> misc_feature
<222> (1)..(1)
<223> 5'- Biotin-(Spacer18)2-

<220>
<221> misc_feature
<222> (1)..(6)
<223> L-RNA

<400> 6
cgcagagcc 9

<210> 7
<211> 40
<212> RNA
<213> Artificial sequence

<220>
<223> Chemically synthesised

<220>
<221> misc_feature
<222> (1)..(40)
<223> L-RNA

<400> 7
gcacgucccu caccggugca agugaagccg uggcucugcg 40

<210> 8
<211> 40

<212>  RNA
<213>  Artificial sequence

<220>
<223>  Chemically synthesised

<220>
<221>  misc_feature
<222>  (1)..(40)
<223>  L-RNA


<220>
<221>  misc_feature
<222>  (1)..(1)
<223>  5'40 kDa-PEG -


<400>  8
gcacgucccu caccggugca agugaagccg uggcucugcg                              40


<210>  9
<211>  40
<212>  RNA
<213>  Artificial sequence

<220>
<223>  Chemically synthesised

<220>
<221>  misc_feature
<222>  (1)..(40)
<223>  L-RNA


<220>
<221>  misc_feature
<222>  (40)..(40)
<223>  -3'40 kDa-PEG


<400>  9
gcacgucccu caccggugca agugaagccg uggcucugcg                              40


<210>  10
<211>  11
<212>  DNA
<213>  Artificial sequence

<220>
<223>  Chemically synthesised

<220>
<221>  misc_feature
<222>  (11)..(11)
<223>  -(Spacer18)2-NH2 -3'

<220>
<221>  misc_feature
<222>  (1)..(11)
<223>  L-DNA


<400>  10
gagggacgtg c                                                          11


<210>  11
<211>  9
<212>  RNA
<213>  Artificial sequence

<220>
<223>  Chemically synthesised

<220>
<221>  misc_feature
<222>  (1)..(1)
<223>  5'- Biotin-(Spacer18)2-


<220>
<221>  misc_feature
<222>  (1)..(9)
<223>  L-RNA


<400>  11
cgcagagcc                                                              9


<210>  12
<211>  45
<212>  RNA
<213>  Artificial sequence

<220>
<223>  Chemically synthesised

<220>
<221>  misc_feature
<222>  (1)..(45)
<223>  L-RNA


<400>  12
gcguggugug aucuagaugu auuggcugau ccuagucagg uacgc                     45


<210>  13
<211>  45
<212>  RNA
<213>  Artificial sequence

<220>
<223>  Chemically synthesised

<220>
<221>  misc_feature
<222>  (1)..(45)
<223>  L-RNA


<220>
<221>  misc_feature
<222>  (1)..(1)
<223>  5'-40 kDa-PEG-


<400>  13
gcguggugug aucuagaugu auuggcugau ccuagucagg uacgc                    45


<210>  14
<211>  45
<212>  RNA
<213>  Artificial sequence

<220>
<223>  Chemically synthesised

<220>
<221>  misc_feature
<222>  (1)..(45)
<223>  L-RNA


<220>
<221>  misc_feature
<222>  (45)..(45)
<223>  -3'-40 kDa-PEG


<400>  14
gcguggugug aucuagaugu auuggcugau ccuagucagg uacgc                    45


<210>  15
<211>  13
<212>  DNA
<213>  Artificial sequence

<220>
<223>  Chemically synthesised

<220>
<221>  misc_feature
<222>  (13)..(13)
<223>  -(C18-PEG-spacer)-(C18-PEG-spacer)-NH2-3'


<220>
<221>  misc_feature
<222>  (1)..(13)
<223>  L-DNA

<400>  15
gatcacacca cgc                                                          13

<210>  16
<211>  11
<212>  RNA
<213>  Artificial sequence

<220>
<223>  Chemically synthesised

<220>
<221>  misc_feature
<222>  (1)..(1)
<223>  5'-Biotin-(C18-PEG-spacer)-(C18-PEG-spacer)-

<220>
<221>  misc_feature
<222>  (1)..(11)
<223>  L-RNA

<400>  16
gcguaccuga c                                                            11